# EUROPEAN PATENT APPLICATION

(11) **EP 4 241 774 A1**
(43) Date of publication of application: **13.09.2023**
(21) Application number: 21889571.2
(22) Date of filing: 04.11.2021
(51) Int. Cl.: A61K 31/5415, A61P 25/00, A61P 25/28, A23L 33/10

(54) **COMPOSITION FOR PREVENTING OR TREATING NEUROINFLAMMATORY DISEASES, COMPRISING CHLORPROMAZINE**

(30) Priority: 04.11.2020 KR 20200146060; 03.11.2021 KR 20210150049
(71) Applicant: Gliacelltech Inc., Seoul 08595 (KR); Daegu Gyeongbuk Institute Of Science and Technology, Daegu 42988 (KR)
(72) Inventor: YU, Seong-Woon, Dalseong-gun, Daegu 42988 (KR); NAM, Hyeri, Dalseong-gun, Daegu 43018 (KR); LEE, Younghwan, Seoul 07071 (KR)
(74) Representative: J A Kemp LLP
(86) International application number: PCT/KR2021/015855
(87) International publication number: WO 2022/098106

(57) **Abstract**

Disclosed herein is a composition for prevention or treatment of neuroinflammatory disease, and more specifically, a use of the composition comprising chlorpromazine or a salt thereof for preventing, improvement, or treating neuroinflammatory diseases.

## Description

### Technical Field

The present disclosure relates to a composition for prevention or treatment of neuroinflammatory disease and, more specifically, to a composition comprising chlorpromazine or a salt thereof for prevention or treatment of neuroinflammatory disease.

### Background Art

Neuroinflammation, which is a kind of immune response in the nervous system, is very closely correlated with many neurodegenerative diseases including Alzheimer's disease, Parkinson's disease, and multiple sclerosis and is currently considered as a hallmark of neurodegenerative diseases. Neuroinflammation includes activation of innate immune cells (microglia), release of inflammatory mediators, e.g., nitric oxide (NO), cytokines, and chemokines, and infiltration of macrophages, inducing death of neural cells. The inflammatory activation of microglia and astrocytes is considered to be an important mechanism for progression of neurodegenerative disease as important pathological markers. Because stringent regulation of the microglial activation is indispensable for maintenance of brain homeostasis and prevention of infection and inflammatory disease, there is a need for development of a substance that can regulate the activity of microglia to palliate neuroinflammation.

Alzheimer's disease is a neuroinflammatory disease that appears in the form of dementia, and dementia is a disorder with general failure of mental functions such as memory impairment, loss of cognition, etc. Dementia manifests in various types, with about 50 % of cases of dementia accounted for by Alzheimer's disease type, about 30 % by vascular-type, alcoholic-type, and Parkinson's disease type dementia, and about 20 % by mixed types including Alzheimer and vascular types. Most cases of Alzheimer type dementia are sporadic with no special genetic factors, some cases are classified as familial Alzheimer's disease, which is attributed to mutations in amyloid precursor protein (APP), presenilin 1 (Psen1), and Psen2 genes.

Due to its nature, Alzheimer's disease has physical and psychological impacts, not only for the patients, but also for their families at large. Alzheimer's disease occurs in 10% of people 65-74 years old, 19% in people 75-84 years old, and 47% in people 85 years old or older, with the incidence increasing every year. As of 1995 in Korea, the number of diagnosed Alzheimer's disease patients over 65 years of age was 241,000, accounting for 8.3% of the total elderly population, and it is expected to reach 619,000 by 2020, which is emerging as a major social problem. There is no known prophylactic method for Alzheimer's disease, nor an established early diagnosis method. Nothing has been developed as therapeutic agents so far, but only drugs that palliate symptoms are being used.

Chlorpromazine, which is one of the phenothiazines, is known as the first-generation drug, synthesized by Rhone-Poulenc Lab in 1950s, for schizophrenia. Targeting a cerebral region responsible for controlling emotions and vomiting, the drug acts to regulate the production and secretion of the neurotransmitters which are generated upon neuronal hyperexcitation, such as dopamine, serotonin, and the like, thereby suppressing excitation and relieving anxiety, tension, apprehension, nausea, vomiting, etc. It is known that there is a side effect of increasing the mortality rate in the elderly with schizophrenia when taken for a long time at the current market dose.

### Disclosure

### Technical Problem

In order to solve the problems encountered in the related art, the present disclosure aims to provide a pharmaceutical composition comprising chlorpromazine, a derivative thereof, a metabolite thereof, or a salt thereof, for prevention, improvement, or treatment of a neuroinflammatory disease.

An additional aspect of the present disclosure is drawn to a method for prevention or treatment of a neuroinflammatory disease, comprising a step of administering a pharmaceutical composition comprising chlorpromazine, a derivative thereof, a metabolite thereof, or a salt thereof to a subject in need thereof.

A further aspect of the present disclosure pertains to chlorpromazine or a pharmaceutically acceptable salt thereof for use in preventing or treating a neuroinflammatory disease in a subject.

In an embodiment of the present disclosure, the composition may regulate the expression of the circadian clock-controlled neuroinflammatory cytokines interleuckine-6 (IL-6), CXCL1, CCL2, and CCL5. In another embodiment of the present disclosure, the composition may recover memory by suppressing neuroinflammation.

### Technical Solution

The present disclosure provides a pharmaceutical composition and a health functional food composition, each comprising chlorpromazine, a derivative thereof, a metabolite thereof, or a salt thereof, for prevention, improvement, or treatment of neuroinflammatory disease.

As used herein, the term "chlorpromazine" (CPZ) refers to the compound with the chemical formula of C₁₇H₁₉ClN₂S and the structure of the following Chemical Formula I, named 3-(2-chloro-10H-phenothiazin-10-yl)-N,N-dimethyl-propan-1-amine according to the IUPAC nomenclature. An active ingredient in the pharmaceutical composition for prevention, improvement, or treatment of neuroinflammatory disease according to the present disclosure may be at least one selected from the group consisting of chlorpromazine, a derivative thereof, a metabolite thereof, and a pharmaceutically acceptable salt thereof.

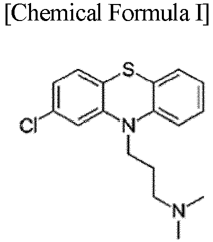

Chlorpromazine undergoes extensive metabolic transformation into a number of metabolites that may be therapeutically active. As such, the metabolites may be substituted for chlorpromazine in neuroinflammatory diseases of the present disclosure. The metabolism of chlorpromazine includes, for example, oxidative N-demethylation to yield the corresponding primary and secondary amine, aromatic oxidation to yield a phenol, N-oxidation to yield the N-oxide, S-oxidation to yield the sulfoxide or sulfone, oxidative deamination of the aminopropyl side chain to yield the phenothiazine nuclei, and glucuronidation of the phenolic hydroxy groups and tertiary amino group to yield a quaternary ammonium glucuronide.

The chlorpromazine according to the present disclosure may be used in the form of pharmaceutically acceptable salts. These salts may be acid addition salts formed with various pharmaceutically acceptable organic or inorganic salts. Acid addition salts may be obtained inorganic acids such as hydrochloric acid, nitric acid, phosphoric acid, sulfuric acid, hydrobromic acid, hydroiodic acid, nitrous acid or phosphorous acid, and nontoxic organic acids such as aliphatic mono- and dicarboxylate, phenyl-substituted alkanoate, hydroxyalkanoate and alkanedioate, aromatic acids, aliphatic and aromatic sulfonic acids. Such nontoxic salts may be prepared using sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butene-1,4-dioate, hexane-1,6-dioate, benzoate, chlorobenzoate, methyl benzoate, dinitrobenzoate, hydroxybenzoate, methoxybenzoate, phthalate, terephthalate, benzenesulfonate, toluenesulfonate, chlorobenzenesulfonate, xylenesulfonate, phenyl acetate, phenyl propionate, phenyl butyrate, citrate, lactate, β-hydroxybutyrate, glycolate, malate, tartrate, methanesulfonate, propanesulfonate, naphthalene-1-sulfonate, naphthalene-2-sulfonate, mandelate, or trifluoroacetate.

An acid addition salt of chlorpromazine can be prepared using a typical method, for example, by dissolving chlorpromazine in an excess of aqueous acid solution and precipitating the salt in a water-miscible organic solvent such as methanol, ethanol, acetone, or acetonitrile. Alternatively, it can be prepared by evaporating the solvent or the excessive acid from the mixture and drying, or by suction filtration of the precipitated salt.

Chlorpromazine may be prepared into a pharmaceutically acceptable salt by using a base. An alkali metal salt or an alkaline earth metal salt can be obtained, for example, by dissolving the compound in an excess of an alkali metal hydroxide solution or an alkaline earth metal hydroxide solution, filtering out the non-soluble compound salt, and evaporating, drying the filtrate. Here, agrichemically suitable metal salts include lithium, sodium, potassium, or calcium salts. In addition, corresponding silver salts can be obtained by reacting the alkali metal or alkaline earth metal salts with a suitable silver salt (e.g., silver nitrate).

As used herein, the term "neuroinflammatory disease" is intended to encompass all the diseases caused by inflammation of the nervous system, and specifically comprise neuroinflammatory diseases caused by overexpression of circadian clock-controlled cytokines, compared to normal subjects.

Examples of the disease comprise multiple sclerosis, neuroblastoma, stroke, Alzheimer's disease, Parkinson's disease, Lou Gehrig's disease, Huntington's disease, Creutzfeldt-Jakob disease, post-traumatic stress disorder, depression, schizophrenia, neuropathic pain, and amyotrophic lateral sclerosis, but are not limited thereto.

The diseases may be cognitive impairment, memory loss, Alzheimer's disease, and related symptoms caused by neuroinflammation induction, and may occur or worsen with aging, genetic mutation, head injury, depression, or complications caused by hypertension. Alzheimer's disease (AD) comprises, for example, sporadic Alzheimer disease (SAD) or familial Alzheimer's disease (FAD). One of the main mutations causing FAD is by mutations of the PSEN gene which is a gene expressing transmembrane protein presenilin comprising the catalytic subunit of gamma-secretase.

As used herein, the term "neuroinflammation-induced Alzheimer's disease" means dementia induced by artificially generating a neuroinflammatory response. This dementia can be established within a short period of time, unlike senescence-associated dementia models. As used herein, the term "neuroinflammation-induced Alzheimer's disease" means dementia induced by artificially generating a neuroinflammatory response.

More specifically, the subject and/or disease according to the present disclosure may be characterized by at least one selected from the group consisting of overexpression of a circadian clock-controlled-cytokine encoding gene, downregulated expression of Nr1d1 (Nuclear Receptor Subfamily 1 Group D Member 1) gene, and mutation of presenilin 2 gene.

The subject and/or disease may be characterized by overexpression of a circadian clock-controlled inflammatory cytokine, compared to normal subjects.

In addition, the subject and/or disease exhibits overexpression of a circadian clock-controlled inflammatory cytokine in glial cells in the central nervous system, compared to normal subjects or normal disease. The glial cells in the central nervous system may comprise radial glia, astrocytes, oligodendrocytes, oligodendrocyte progenitor cells, and microglia. Specifically, the glia cells in the central nervous system may be microglia and astrocytes and more specifically microglia.

The circadian clock-controlled inflammatory cytokine is at least one selected from the group consisting of IL-6, CXCL1, CCL2, and CCL5.

Specifically, the present inventors found that application of chlorpromazine to microglia in a mouse model of a neuroinflammatory disease, for example, neuroinflammation-induced Alzheimer's disease repressed the expression of clock-controlled inflammatory cytokines, thereby exhibiting an anti-inflammatory effect and a memory recovery effect.

The subject and/or disease may be characterized by a lower expression level of Nr1d1 gene, compared to normal subjects or normal diseases. In detail, the low expression level of Nr1d1 gene or its protein (REV-ERBα) may induce the overexpression of the circadian clock-controlled cytokines.

The subject and/or disease may have PSEN2 gene mutation, unlike normal subjects or other diseases. Specifically, in the subject and/or disease, the Nr1d1 gene or protein may be decreased or suppressed by the mutation of PSEN2 gene, and overexpression of a circadian clock-controlled cytokine may be induced by a decreased or suppressed expression of Nr1d1 gene or protein.

Presenilin (PS) constitutes a catalytic subunit of the membrane-bound γ-secretase complex that cleaves amyloid-β protein precursor (AβPP) to produce an amyloid-β (Aβ) peptide. Mutations on presenilin 2 gene (PSEN2) accounts for less than 5% of all the cases of early-onset familial Alzheimer's disease (EOFAD).

Early-onset familial Alzheimer's disease (EOFAD) is known to be caused by heterozygous variant of presenilin 1 (PSEN1), presenilin 2 (PSEN2), and APP gene. The mechanism how these mutated genes cause Alzheimer's disease (AD) and accelerates the progression of AD remains unclear. It is known that the enzymatic activity of presenilin (PS) is responsible for the production of amyloid-β (Aβ).

As used herein, the term "PSEN2 gene" refers to a gene coding for the PSEN2 polypeptide. Based on the NCBI reference sequence, the PSEN2 gene is located at NC_000001.11 (226870594..226903829) in humans, at NC_000067.7 in mice, and comprises known orthologs as well as those sequences.

Mutations on the PSEN2 gene, associated with neuroinflammation and/or Alzheimer's disease, comprise A85V, N141I, N141Y, M174I, G212V, A237V, M239I, or M239V (Jiang et al., "A Review of the Familial Alzheimer's Disease Locus PRESENILIN 2 and Its Relationship to PRESENILIN 1." Journal of Alzheimer's Disease 66 (2018) 1223-1339). At least one of the mutations may be comprised in the present disclosure.

Leading to the present disclosure, intensive and thorough research conducted by the present inventors resulted in the finding that when treated with chlorpromazine, Psen2^{N141I} knock-in (KI) animal models possessing the substitution of I (isoleucine) for N (arginine) at position 141 on presenilin 2 gene, which is one of the mutations responsible for familial Alzheimer's disease, has repressed expression levels of the circadian clock-controlled cytokines IL-6 (interleuckine-6), CXCL1, CCL2, and CCL5, whereby an anti-inflammatory effect and a memory recovery effect was observed in the animal models.

The composition according to the present disclosure may have at least one of the features of suppressing neuroinflammation, suppressing the overexpression of a circadian clock-controlled cytokine, recovering REV-ERVα expression, recovering memory, and improving cognitive performance, which brings about prevention, improvement, or treatment of neuroinflammatory diseases. In greater detail, the composition according to the present disclosure was found to suppress the expression of neuroinflammatory cytokines to exhibit a prophylactic, improvement, or therapeutic effect on Alzheimer's disease as assayed for memory recovery and anti-inflammatory activity in animal models.

As used herein, the term "prevention" refers to any action of inhibiting the progression of neuroinflammatory disease or delaying the onset thereof by administering the composition according to the present invention. As used herein, the term "improvement" refers to any action of reducing the severity of symptom of neuroinflammatory disease by administering the composition according to the present invention.

The term "treatment", as used herein, refers to any action in which symptoms of neuroinflammatory disease are improved or beneficially changed by administering the composition according to the present disclosure. In detail, the term "treatment", as used herein, is intended to encompass the palliation or reduction of at least one symptom associated with or caused by a state, disorder, or disease under therapy. A treated subject may exhibit partial or total alleviation of symptoms (for example, Alzheimer's disease or an associated condition) or symptoms may remain static after treatment according to the present disclosure. The term "treatment" is intended to comprise prevention, therapy and cure.

More specifically, it was observed that treatment with chlorpromazine restored the expression of REV-ERBα and exhibit immunosuppression and a prophylactic effect on cognitive decline in animal models with Alzheimer's disease (Psen2^{N141I} KI/+ mice). The composition of the present disclosure can restore memory by suppressing neuroinflammation through mechanisms such as downregulated expression of the cytokines, etc.

In an embodiment of the present disclosure, it was observed that microglia in Alzheimer's disease-induced animal models expressed high levels of the circadian clock-controlled inflammatory cytokines IL-6, CXCL1, CCL2, and CCL5 and the secretion and expression of the circadian clock-controlled inflammatory cytokines was effectively decreased by treatment with chlorpromazine. In addition, pretreatment with chlorpromazine in advance of LPS injection decreased blood levels of the circadian clock-controlled inflammatory cytokines. Also, as assayed by a Y-maze test, chlorpromazine was observed to rescue memory deficits by recovering the arm alternation in the animal model to a level similar to that in a normal control.

In the present disclosure, the substitution N141I in Psen2, known to cause familial AD (FAD) in humans, was conducted in mice by homologous recombination to induce dementia through inducing neuroinflammation, whereby a mouse model of definite dementia could be constructed. The animal model of Alzheimer's disease, resulting from replacement of wild-type Psen2 gene by Psen2^{N141I}, were used for confirming efficacy and concentration of chlorpromazine. According to the present disclosure, the animal model may be obtained by a method comprising the steps of (a) inserting a Psen2^{N141I} mutation gene into a targeting vector; (b) introducing the gene-inserted vector into a host animal; and (c) crossing the vector-introduced host animal with Cre mice to obtain descendant animals.

The animal model which has been made to suffer from the same mutation as that reported for humans by conducting a substitution mutation in the wild-type Psen2 gene and into which the same heterozygous mutation as in Alzheimer's disease patients has been introduced to maintain the endogenous expression level of the mutated gene, whereby the model can more accurately be modelling human Alzheimer's disease or relevant dementia and can be utilized for precise analysis for dementia pathogenesis, compared to conventional transgenic animal models in which the FAD human gene is overexpressed in addition to the expression of the normal gene of the animal itself. For use in assaying the efficacy of chlorpromazine, the animal model was intraperitoneally injected with lipopolysaccharide (LPS) or microglia were treated with LPS or fibrilized amyloid beta 1-42 (fAβ42), which is considered a direct trigger of Alzheimer's disease, to induce neuroinflammation through the expression of inflammatory cytokines, for example, Alzheimer's disease.

When the composition according to the present disclosure is in a form of a pharmaceutical composition, it may contain a pharmaceutically effective amount of chlorpromazine alone or in combination with at least one pharmaceutically acceptable carrier.

Currently, the chlorpromazine and the like according to the present disclosure may be obtained in the form of a tablet, a capsule, a suppository, an oral concentration, a syrup, and an injectable formulation.

So long as it is typically available for formulations, any pharmaceutical acceptable carrier may be used in the present disclosure. Examples of the pharmaceutically acceptable carrier include, but are not limited to, lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxybenzoate, propyl hydroxybenzoate, talc, magnesium stearate, and mineral oil. In addition to the ingredients, the composition may further comprise a lubricant, a humectant, a sweetener, a flavorant, an emulsifier, a suspending agent, a preservative, and the like.

According to intended methods, the pharmaceutical composition of the present disclosure may be administered orally or parenterally (e.g., intravenously, subcutaneously, intraperitoneally, or topically). The dose of the composition may vary depending on the condition and weight of the patient, the severity of disease, dosage form, administration routes, and the duration of administration, and may be suitably selected by a person skilled in the art.

As used herein, the term "individual", "subject", or "patient" may be mammals such as rats, livestock, mice, humans, and so on and particularly, companion dogs, racehorses, and humans, and so on in need of treatment of a neuroinflammatory disease, and preferably humans.

The pharmaceutical composition of the present disclosure is administered in a pharmaceutically effective amount. As used herein, the term "pharmaceutically effective amount" means an amount necessary for achieving a therapeutical effect. The therapeutic effect could be any therapeutic effect ranging from prevention, symptom amelioration, symptom treatment, to disease termination or cure, for example, cure of Alzheimer's disease or related pathologies.

In the Psen2 mouse model of familial Alzheimer's disease, application of chlorpromazine suppressed the expression of the circadian clock-controlled inflammatory cytokines IL-6, CXCL1, CCL2, and CC5, thereby bringing about an anti-inflammatory effect and memory restoration. In the present disclosure, chlorpromazine was measured to be effective even when used at a much lower concentration than that for clinical use as an antipsychotic medicine.

In an embodiment of the present disclosure, the daily dose of chlorpromazine for oral administration to humans is 10- to 100-fold lower than that adopted as an antipsychotic medicine. In detail, for chlorpromazine free base, the dose may be 1.0 mg /kg/day or less, 0.8 mg /kg/day or less, 0.6 mg /kg/day or less, 0.5 mg /kg/day or less, 0.25 mg /kg/day or less, or 0.1 mg /kg/day or less. The lower limit of the daily dose of chlorpromazine for humans is the minimum amount to achieve a desired efficacy and may be, for example, about 0.00001 to 0.0001 mg/kg/day. By way of example, chlorpromazine may be administered to humans at a dose of about 0.00001 to 1.0 mg /kg/day, or about 0.0001 to 1.0 mg /kg/day, based on chlorpromazine free base. The dosage amount may be administered once or may be divided into multiple doses. It is known that the commercially available antipsychotic drug chlorpromazine, when taken for a long time at the high dose as it is marketed, increase mortality in elderly patients with schizophrenia. With such an effective dose much lower than those of conventional antipsychotic drugs, the chlorpromazine regimen according to the present disclosure is very advantageous.

No particular limitations are imparted to the content of chlorpromazine in the pharmaceutical composition of the present disclosure. The dose may vary depending on the state of a subject to be treated, the type of symptoms, the extent of disease progression, etc. If necessary, the content of chlorpromazine may account for the total amount of the composition.

In an experimental example, wild-type and KI/+ mice were intraperitoneally (i.p.) injected with various concentration of LPS at 18:00 and monitored for inflammatory responses after 20 hours. Compared with WT mice, KI/+ mice exhibited a higher circulating level of II,-6 at all LPS doses tested, and an increased blood levels of circadian clock-controlled cytokines in KI/+ mice were detected at lower concentration of LPS, indicating that a low dose of LPS induces hyperactive immune responses and provokes memory deficits in the Psen2^{N141I}/+ mice through overexpression of the circadian clock-controlled cytokines.

In the present disclosure, an animal model has been constructed to suffer from neuroinflammation-induced dementia by introducing into the wild-type mouse Psen2 gene the substitution N141I, which is reported to cause familial Alzheimer's disease (FAD) in humans, through homologous recombination, whereby the animal model can more accurately be modelling the dementia within a short period of time.

As used herein, the term "homologous recombination" refers to a type of genetic recombination in which genetic information is exchanged between two similar or identical molecules of DNA. In this regard, a vector carrying a mutation may be inserted into a homologous region of a wild-type gene in a host animal. The resultant vector-inserted host animal may be crossed with Cre mice to obtain a descendant as an animal model. In the present disclosure, the vector may include the N141I mutation in exon 4 of Psen2 gene and the Neo^{r}-loxp sequence and mice transduced with the vector may be crossed with Cre mice by means of the Cre-loxp system to generate knock-in mice carrying Psen2^{N141I} mutation.

The substitution was conducted in the wild-type mouse Psen2 gene to make the same mutation as the dementia mutation reported for humans. In addition, heterozygous mutations were introduced as in Alzheimer's disease patients to maintain the endogenous expression level, whereby the model can more accurately be modelling human dementia and can be utilized for precise analysis for dementia pathogenesis. Furthermore, intraperitoneal injection of LPS causes the expression of inflammatory cytokines, leading to the construction of a neuroinflammation-induced Alzheimer's disease model within a short period of time (two months), unlike the dementia models generated with aging. Thus, the model is expected to find high applicability.

In concrete examples of the present disclosure, the animal models were generated and identified to reproduce human Psen2^{N141I} dementia mutation as analyzed for expression of cytokines, circadian clock-controlled genes.

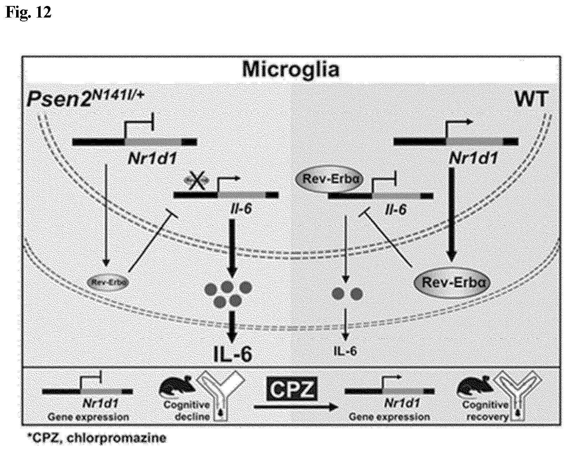

FIG. 12 is a schematic diagram illustrating the mechanism of expression of circadian clock-controlled inflammatory cytokines and a change by chlorpromazine treatment in microglia of wild-type and Psen2^{N141I}/+ AD mouse models.

### Advantageous Effects

In the present disclosure, a Psen2^{N141I} mutation mouse model of familial Alzheimer's disease was constructed and identified to undergo excessive neuroinflammation. Also, chlorpromazine was observed to have an anti-inflammatory activity to inhibit the excessive neuroinflammation, with the recovery of memory in the animal model. Therefore, the present disclosure is expected to contribute to the design of a novel dementia therapy which could be concretely established through further studies for dementia pathogenesis.

### Description of Drawings

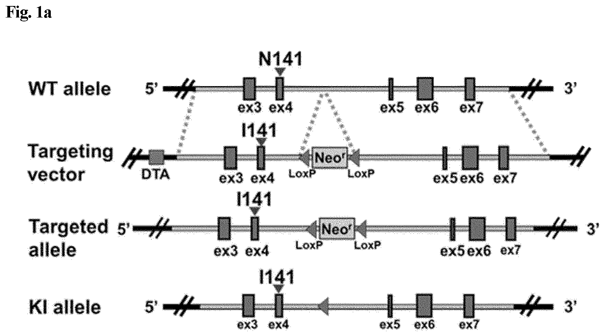

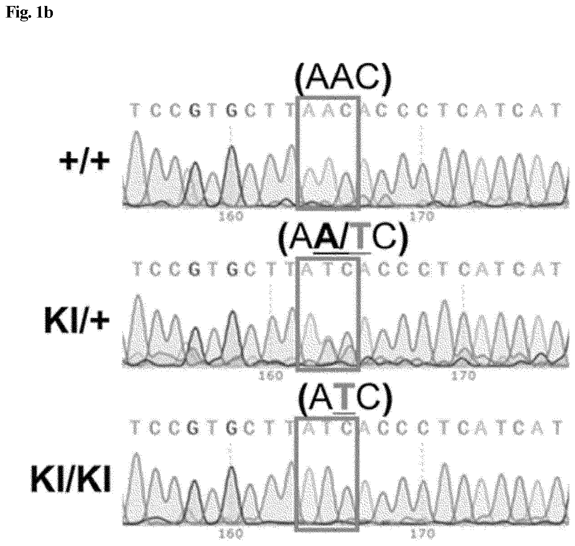

FIG. 1 exhibits construction of a Psen2^{N141I} mouse model of Alzheimer's disease according to an embodiment of the present disclosure: FIG. 1a is a schematic illustration for a strategy for targeted insertion of N141I; and FIG. 1b shows Sanger sequencing chromatograms of normal(wild-type), KI/+, and KI/KI mice.

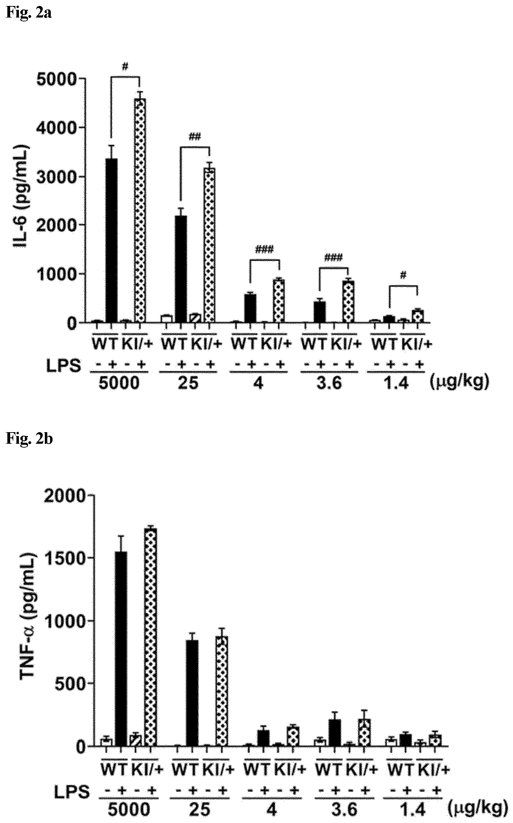

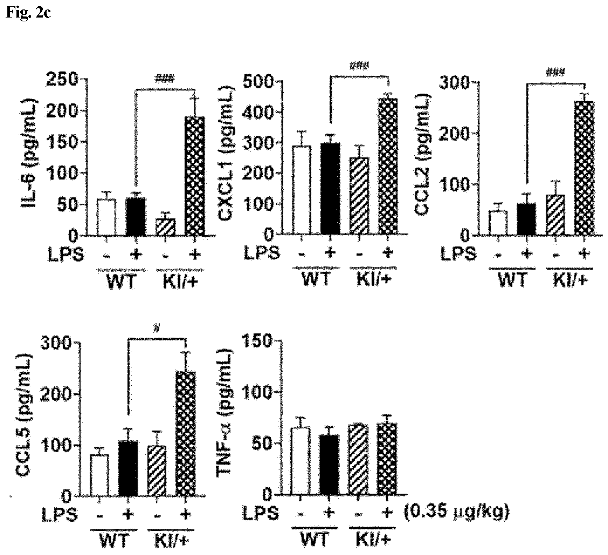

FIG. 2 shows hyperactive inflammatory responses in the Psen2^{N141I} mutant mouse model of Alzheimer's disease according to an embodiment of the present disclosure, compared to normal(wild-type) mice: FIG. 2a shows blood levels of IL-6 in animals into which lipopolysaccharide (LPS) has been intraperitoneally injected at various concentration to induce neuroinflammation. Intraperitoneal injection of LPS into Psen2 mutation Alzheimer's disease mice induced overproduction of IL-6 at all the concentrations. The relative difference between the normal mice and the model of Alzheimer's disease was more pronounced at the lower concentration; FIG. 2b shows the production of TNF-α by intraperitoneal injection of LPS. At all the LPS concentrations, similar blood levels of TNF-α were detected between normal mice and Psen2^{N141I} mutation mice of Alzheimer's disease; and FIG. 2c shows changes in blood levels of CXCL1, CCL2, and CCL5, all known as circadian clock-controlled inflammatory cytokines. Injection of LPS at a low concentration (0.35 µg/kg) ineffective for inflammation induction in wild-type mice significantly increased production of the circadian clock-controlled inflammatory cytokines only in Psen2^{N141I} mutation mice, unlike TNF-α.

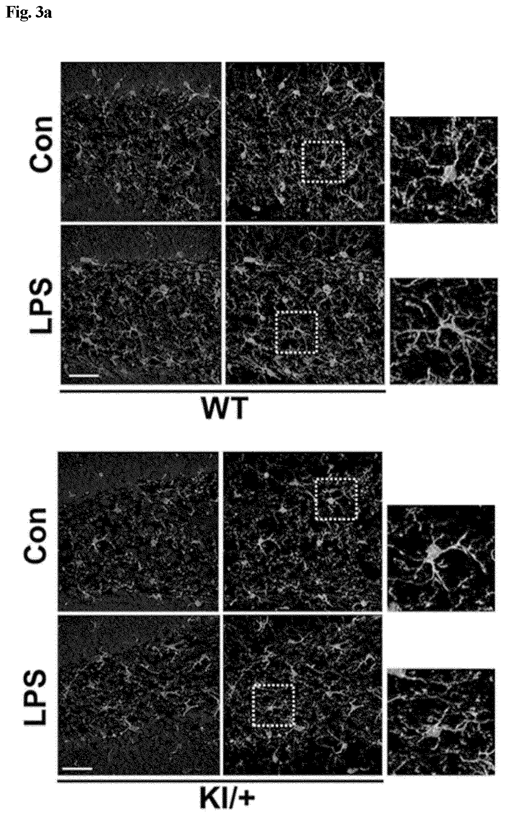

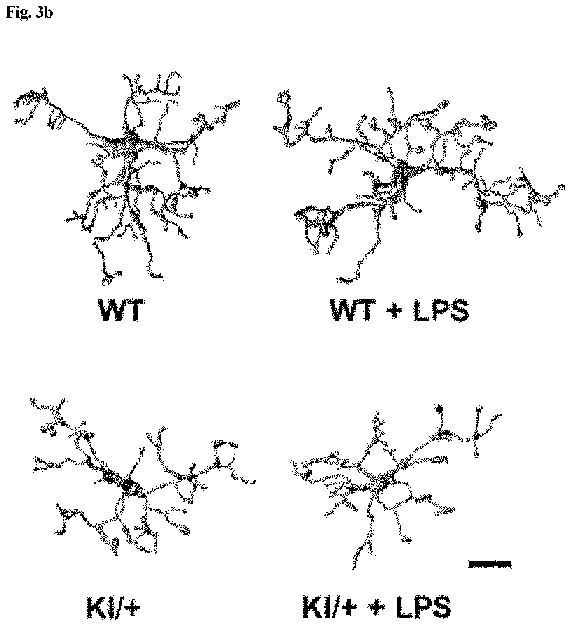

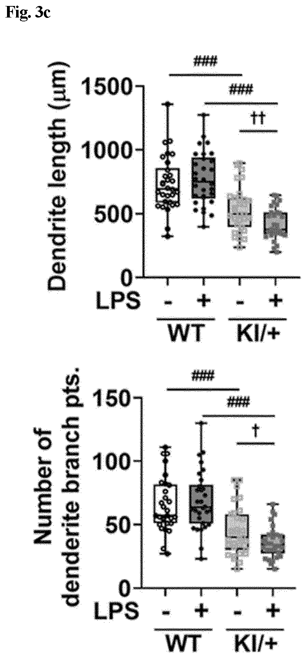

FIG. 3a shows immunofluorescent images by Iba-1 (microglia marker antibody) staining in the hippocampus of normal and Psen2^{N141I} mutant Alzheimer's disease mice; FIG. 3b shows 3D filament tracking images of Iba-1 signals made by IMARIS software; and FIG. 3c shows dendrite lengths and numbers of dendrite branch points as analyzed by FilamentTracker in IMARIS software. From the data, it is understood that Psen2^{N141I} mutant Alzheimer's disease mice produce circadian clock-controlled cytokines in response to even a low inflammatory stimulus to exhibit hyperimmunity.

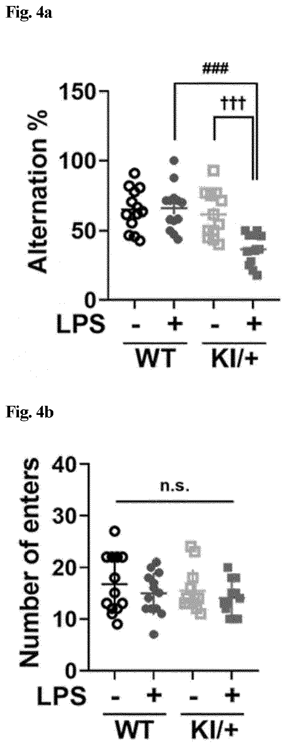

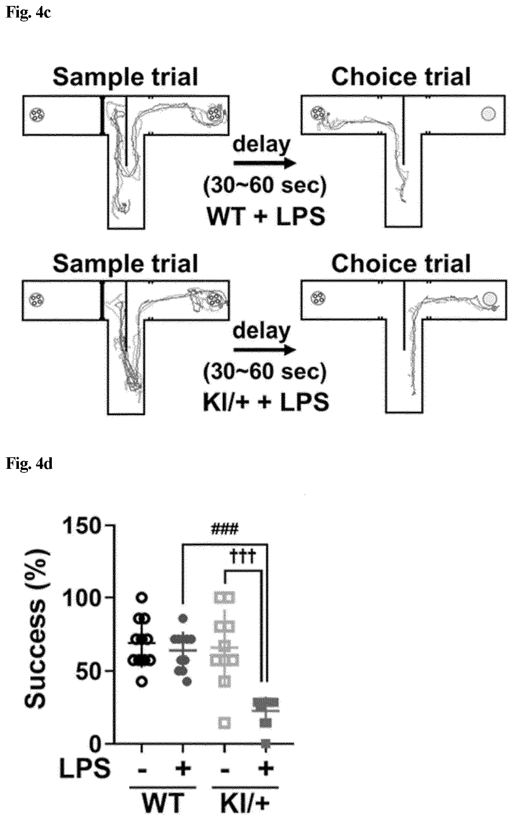

FIG. 4 explains that intraperitoneal injection of LPS at a low concentration induces memory decline in the Psen2^{N141I} mutant mouse model of Alzheimer's disease; FIG. 4a shows the memory deficit of the neuroinflammation-induced Psen2^{N141I} mutant mice as analyzed by the Y-maze test; FIG. 4b shows no difference in locomotive performance between the mice in the Y-maze analysis; FIG. 4c is a schematic diagram for T-maze test procedures; and FIG. 4d shows success rates (%) in T-maze test in which the neuroinflammation-induced Psen2^{N141I} mutant mice are identified to suffer from memory deficits as revealed by the significantly decreased success rates.

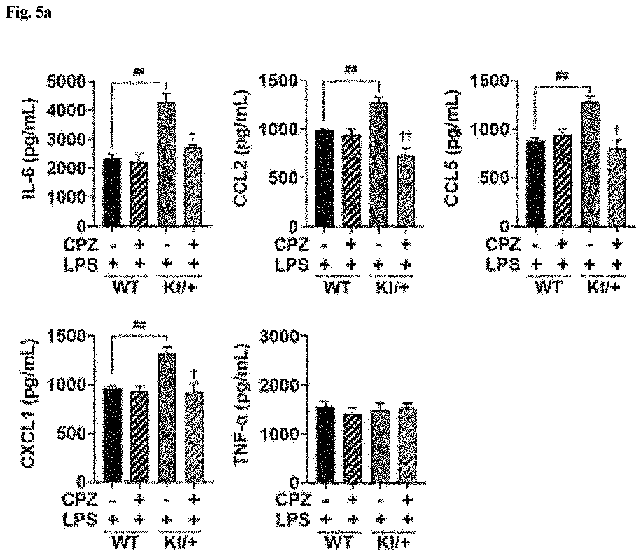

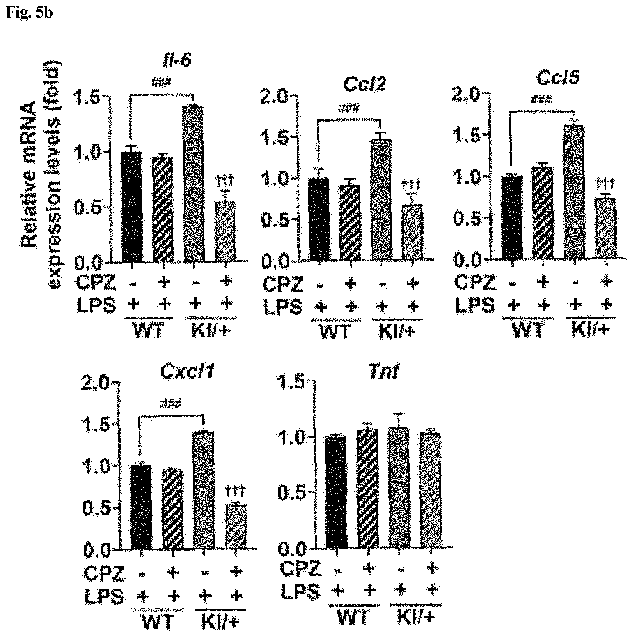

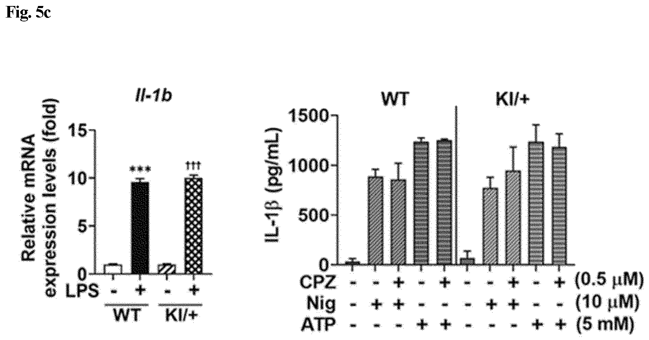

FIG. 5 shows anti-inflammatory effects of chlorpromazine in the Psen2 mutation Alzheimer's disease model in which neuroinflammation has been induced by low concentration of LPS: FIG. 5a shows the suppressive effect of chlorpromazine on the production of the circadian clock-controlled inflammatory cytokines IL-6, CXCL1, CCL2, and CCL5 in primary microglia; FIG. 5b shows the suppressive effect of chlorpromazine on mRNA expression of circadian clock-controlled inflammatory cytokines. As opposed to the circadian clock-controlled cytokines, the production and mRNA expression of TNF-α is not affected by chlorpromazine; and FIG. 5c shows that LPS induces the production and mRNA expression of IL-1β, known as a circadian cycle-independent cytokine, at similar levels between normal cells and Psen2^{N141I} mutant microglia, with no impacts of chlorpromazine on the production of IL-1β across all the groups.

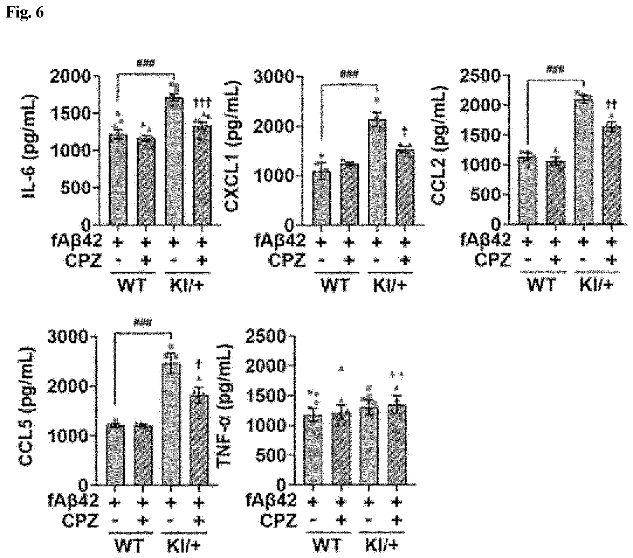

FIG. 6 shows that treatment with fibrilized amyloid beta 1-42 (fAβ42), which is a direct etiological factor of Alzheimer's disease, instead of LPS, remarkably increases production of the circadian clock-controlled inflammatory cytokines IL-6, CXCL1, CCL2, and CCL5 only in the Psen2^{N141I} mutant microglia, and chlorpromazine inhibits the production thereof. In contrast, TNF-α is produced at similar levels between normal microglia and Psen2^{N141I} mutant microglia, with no impacts of chlorpromazine thereon.

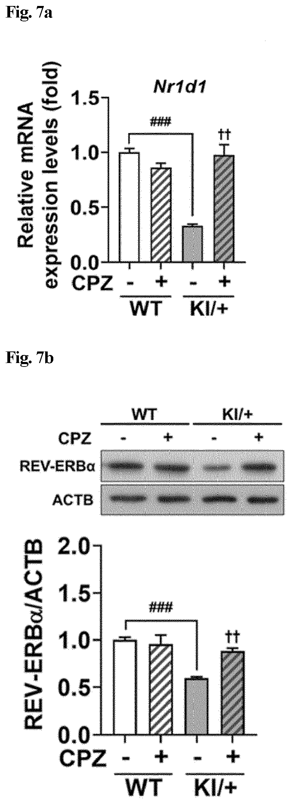

FIG. 7a shows that chlorpromazine restores mRNA expression of the *Nrldl* gene coding for REV-ERBα in the microglia wherein the mRNA expression level has been downregulated by Psen2^{N141I} mutation; and FIG. 7b shows that chlorpromazine restores expression of REV-ERBα protein.

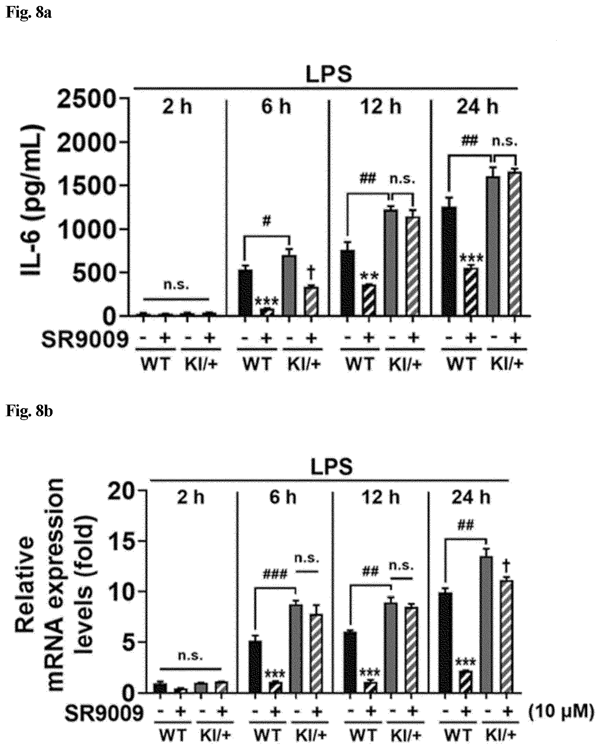

FIG. 8 shows an anti-inflammatory effect of SR9009 drug known as a REV-ERBα agonist, in which SR9009 inhibits the protein expression level (FIG. 8a) and mRNA expression level (FIG. 8b) of IL-6 in normal microglia over time, but cannot inhibit LPS-induced overexpression of Il-6 in Psen2^{N141I} mutant microglia. From the data, it can be seen that the restoration of REV-ERBα expression must be preceded in order to suppress the excessive neuroinflammatory response caused by the Psen2^{N141I} mutation. Also, the data suggest that the REV-ERBα agonist, although exhibiting anti-inflammatory effects in normal microglia, may not have any therapy effect for Psen2 familial dementia.

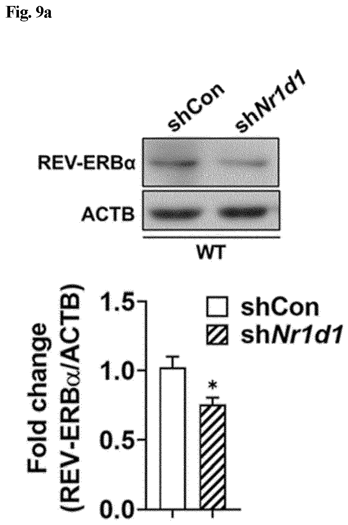

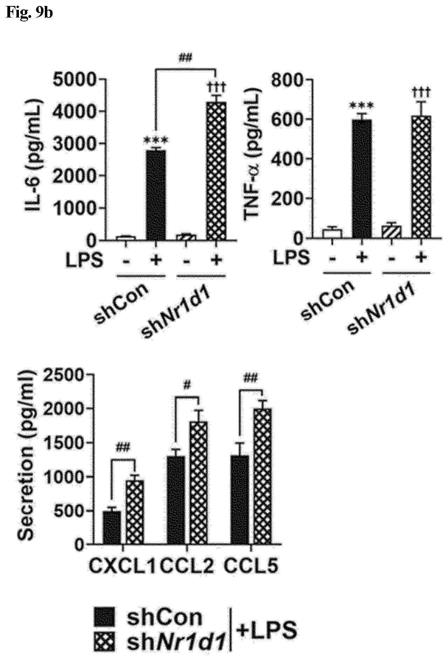

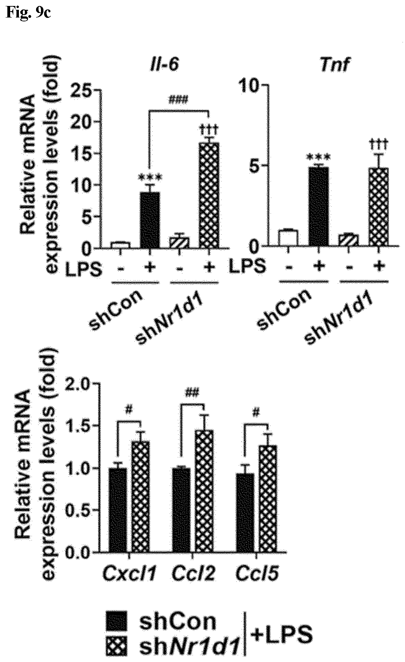

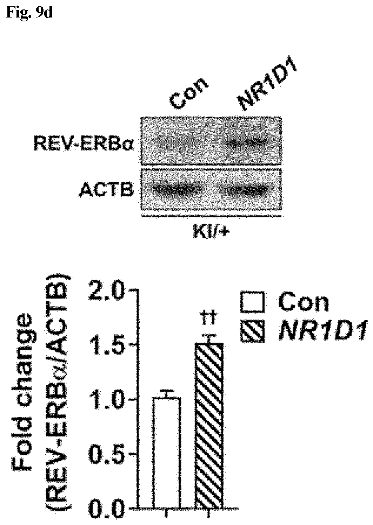

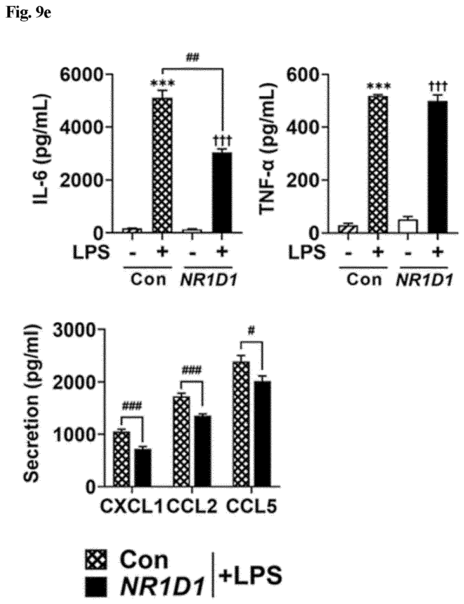

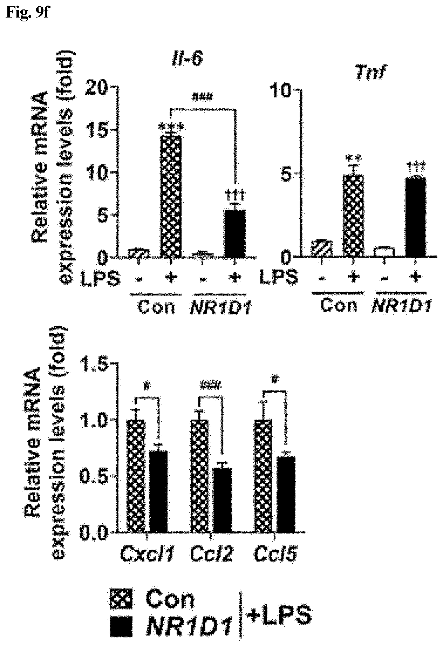

FIG. 9 shows data from experiments designed to explain that downregulated expression of REV-ERBα provokes overexpression of the circadian clock-controlled cytokines: FIG. 9a shows the successful knockdown of REV-ERBα in normal microglia, FIG. 9b shows that REV-ERBα knockdown elevates the production of the cytokines IL-6, CXCL1, CCL2, and CCL5 by LPS treatment; FIG. 9c shows that REV-ERBα knockdown elevates the mRNA expression of the cytokines IL-6, CXCL1, CCL2, and CCL5 by LPS treatment; FIG. 9d shows overexpression of REV-ERBα in primary Psen2^{N141I} mutant microglia; FIG. 9e shows that overexpression of REV-ERBα reduces the LPS-induced production of IL-6, CXCL1, CCL2, and CCL5; and FIG. 9f shows that overexpression of REV-ERBα remarkably reduces the LPS-induced mRNA expression of IL-6, CXCL1, CCL2, and CCL5. In contrast, TNF-α, known as a circadian cycle-independent cytokine is not affected by the regulated expression of REV-ERBα. The data indicate that the downregulated expression of REV-ERBα provokes overexpression of the circadian clock-controlled cytokines in Psen2^{N141I} mutant microglia.

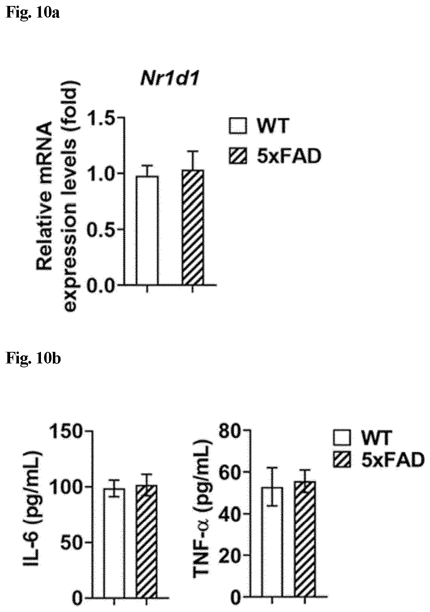

FIG. 10a shows similar *Nrldl* mRNA expression levels between microglia from the wild-type mice and the mouse model of 5xFAD Alzheimer's disease; FIG. 10b shows that there is no difference in blood levels of inflammatory cytokines IL-6 and TNF-α in the mouse model of 5xFAD Alzheimer's disease. The 5xFAD mouse model expresses *APP* and *PSEN1* genes with a total of 5 AD-related mutations (APP; Swedish (K670N/M671L), Florida (I716V), and London (V717I) mutations and PSEN1; M146L and L286V mutations). These data indicate that the hyperactive immune response due to the downregulated expression of REV-ERBα may be responsible for a feature of the Psen2 familial Alzheimer's disease.

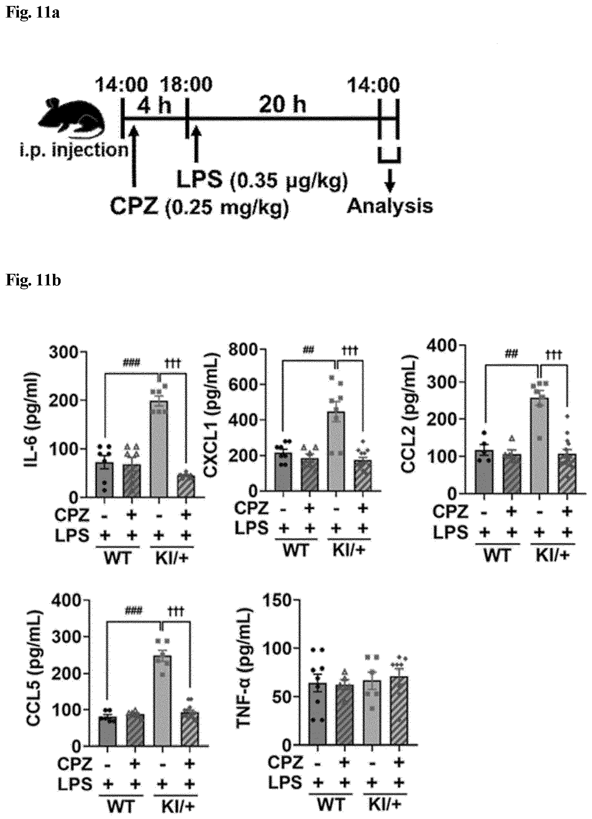

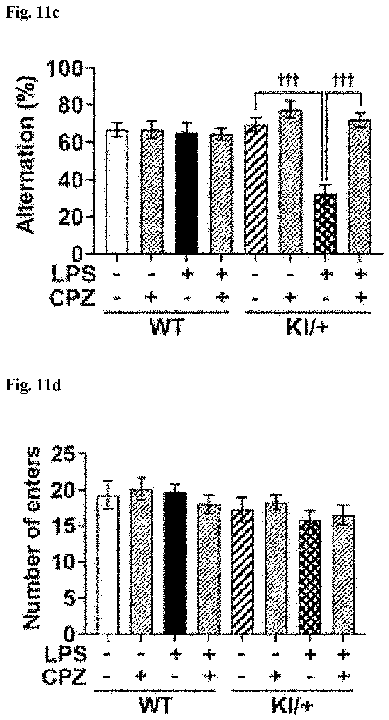

FIG. 11 shows data from in vivo experiments designed to examine the effect of chlorpromazine on the expression of circadian clock-controlled inflammatory cytokines and memory restoration; FIG. 11a is a schematic diagram recapitulating the experiment strategy. The dose of chlorpromazine (0.25 mg/body weight kg) used in this assay is tens- to hundreds-fold lower than those conventionally used as an antipsychotic (25 mg/injection, 3 times/day); FIG. 11b shows blood cytokine levels after the experiment conducted according to the strategy of FIG. 11; FIG. 11c shows the effect of chlorpromazine on memory restoration in neuroinflammation-induced mice as analyzed by the Y-maze test; and FIG. 11d shows no differences in locomotor performance across the mice as measured by the Y-maze test.
FIG. 12 is a schematic diagram illustrating the mechanism of expression of inflammatory cytokines and a change by chlorpromazine treatment in microglia of normal control and neuroinflammatory Alzheimer animal models.

### Mode for Invention

A better understanding of the present disclosure may be obtained via the following examples which are set forth to illustrate, but are not to be construed as limiting the present disclosure.

### EXAMPLE 1: Construction of animal model of disease

All protocols for the care and use of animals were approved by and in accordance with the guidelines established by the Institutional Animal Care and Use Committee of DGIST. Animals were maintained in a specific pathogen-free environment under a 12-hour light/12-hour dark cycle at the DGIST animal facility.

In order to more accurately reproduce a human neuroinflammatory disease, for example, Alzheimer's disease and maintain the endogenous expression level, heterozygous Psen2^{N141I}/+ (KI/+) mice were used. The Psen2^{N141I}/+ mice were generated using homologous recombination.

Specifically, construction was made of a Psen2^{N141I} knock-in (KI) animal model in which the amino acid N (arginine) at position 141 in the presenilin 2 gene is substituted by I (isoleucine), which is one of the mutations responsible for the familial Alzheimer's disease. A targeting vector included the I141 mutation in exon 4 region and the Neo^{r}-loxp sequence, and a homologous region in the targeting vector was inserted into Psen2 of the wild-type (WT) allele. Using the Cre-loxp system, Psen2^{N141I/N141I};loxp-Neo^{r}-loxp mice were crossed with Cre mice to generate Psen2^{N141I} mutant knock-in mice.

In this example, "Psen2^{N141I}" means that the normal psen2 gene of animal model is substituted to express the same mutation as the dementia mutation reported in humans, and more specifically, amino acid 141 of the mouse presenilin 2 gene is substituted from N to I. In the present disclosure, the Psen2^{N141I} gene has the polynucleotide of SEQ ID NO: 1 and the sequence of wild-type Psen2 gene is represented by SEQ ID NO: 2.

As shown in FIG. 1a, KI mice carrying Psen2^{N141I} alleles (Psen2^{N141I/+} and Psen2^{N141I/N141I}) were generated. As shown in FIG. 1b, the nucleotide substitution of the AAC codon for asparagine (N) into ATC codons in one allele generated a KI/+ model carrying both asparagine (N) and isoleucine (I), and the nucleotide substitution of the AAC codon in two alleles produced a KI/KI model carrying two 1141, which was confirmed by genome sequencing for DNA isolated from the tails of the models.

### EXAMPLE 2: LPS-Induced Inflammation in Animal Model of Disease

In this example, an examination was made to show whether Psen2^{N141I} mutant mouse models with Alzheimer's disease underwent excessive inflammation, compared to normal (wild-type) mice.

### 2-1: Assay for LPS concentration to induce inflammation

In order to confirm that Psen2^{N141I}/+ mice suffer from inflammation and cognitive decline as the immune response of the microglia derived therefrom is aggravated, immune responses were compared between wild-type and Psen2^{N141I}/+ mice following injection of various concentrations of LPS thereto.

In mice, the immune response peaks in the hours around the beginning of the active phase. LPS was intraperitoneally (i.p.) injected into 8-week-old wild-type and Psen2^{N141I}/+ mice at 18:00 (Zeitgeber time 11:00, light-on at 07:00 and light-off at 19:00). After 20 hours, they were monitored for the inflammatory response at 14:00 in the next day. LPS, derived from *Escherichia coli* O111:B4 strain, acts as a ligand for toll-like receptor 4 to induce an immune response in cells. Predetermined dilutions of LPS in phosphate-buffered saline (PBS) were each intraperitoneally injected at a concentration of 100 µL to mice.

Specifically, intraperitoneal injection of LPS at concentrations of 1.4, 3.6, 4.0, 25, and 5,000 µg/kg into the Psen2^{N141I} mutation mouse model of Alzheimer's disease induced neuroinflammation.

In a comparison experiment, neuroinflammation induction was made by LPS injection to a wild-type mouse model, instead of the mouse model of Alzheimer's disease. In other comparison experiments, the wild-type mice and the Psen2^{N141I} mutation mouse model of Alzheimer's disease any of which was not injected with LPS were prepared. Thus, the wild-type mice without LPS injection (WT(LPS(-))) were allocated to group 1, the wild-type mice with LPS injection at various concentrations (WT(LPS(+))) to group 2, the mouse model of Alzheimer's disease without LPS injection (KI/+(LPS(-))) to group 3, and the mouse model of Alzheimer's disease with LPS injection at various concentrations (KI/+(LPS(+))) to group 4. Each group consisted of 5-8 mice.

To investigate neuroinflammation at various concentrations of LPS, blood was extracted from cheek veins in the wild-type mice in group 2 and the mouse model of Alzheimer's disease in group 4 20 hours after the injection of LPS. The sera obtained by centrifugation were quantitatively analyzed for IL-6, TNFα, CCL2, CXCL1, and CCL5 by using ELISA kit (R&D Systems) according to the manufacturer's instruction. In addition, the same procedure was conducted for the wild-type mice without LPS injection of group 1 and the Psen2^{N141I} mutation mouse model of Alzheimer's disease of group 3 to obtain sera which were then analyzed for the proteins by using the ELISA kit according to the manufacturer's instruction.

Measurements of IL-6 and TNF-α levels in sera from the mice of groups 1 to 4 are summarized in Table 1, below.

**TABLE 1**

| LPS Concentration (µg/kg) | | 1.4 | 3.6 | 4.0 | 25 | 5000 |
|---|---|---|---|---|---|---|
| Group 1-WT(LPS(-)) | IL-6 (pg/mL) | 55.983± 5.891 | 6.167± 2.833 | 26.815± 6.121 | 152.718± 7.843 | 47.066± 4.710 |
| | TNF-α (pg/mL) | 57.129± 15.581 | 51.606± 16.486 | 13.801± 6.203 | 7.112± 2.436 | 59.262± 18.848 |
| Group 2-WT(LPS(+)) | IL-6 (pg/mL) | 135.414± 12.377 | 435.609± 58.851 | 589.102± 31.891 | 2186.084± 152.786 | 3362.240± 261.326 |
| | TNF-α (pg/mL) | 95.940± 15.385 | 212.951± 57.449 | 127.232± 32.518 | 844.100± 52.867 | 1549.235± 124.825 |
| Group 3-KI/+ (LPS(-)) | IL-6 (pg/mL) | 65.998± 13.677 | 6.167± 2.833 | 14.302± 5.915 | 180.661± 7.031 | 54.023 ±6.36 |
| | TNF-α (pg/mL) | 35.391± 13.406 | 21.775± 9.850 | 19.633± 6.349 | 9.389± 2.097 | 88.176± 18.527 |
| Group 4-KI/+ (LPS(+)) | IL-6 (pg/mL) | 260.651± 28.433 | 858.809± 45.574 | 884.273± 27.475 | 3180.879± 95.747 | 4596.187± 136.528 |
| | TNF-α (pg/mL) | 93.490± 24.912 | 218.504±666.226 | 157.121±13.298 | 874.443±65.026 | 1736.561±19.360 |

In Table 1, the measurements of L-6 and TNF-α levels in blood for each group are expressed as mean ± SEM. The wild-type mice of group 2 and the mouse model of Alzheimer's disease of group 4, which were both injected with various concentrations of LPS, overexpressed IL-6. The difference in expression level between the wild-type mice and the mice model of Alzheimer's disease was relatively increased with the decrease of the concentrations. The blood levels of TNF-α were the same in both the wild-type mice and the mouse model of Alzheimer's disease by LPS. The wild-type mice of group 1 and the Psen2^{N141I} mutation mouse model of Alzheimer's disease of group 3, none of which were injected with LPS, showed very low and similar secretion levels for both IL-6 and TNF-α.

Consequently, as shown in FIG. 2a, the KI/+ mouse model of Alzheimer's disease exhibited a higher circulating level of IL-6 at all LPS concentrations tested, compared with the wild-type mice, and the relative difference between the genotypes was more pronounced at the lower concentration. The blood levels of TNF-α were the same in both genotypes at all concentrations.

### 2-2: Inflammation induction in disease animal by treatment with low concentration of LPS

Animals of groups 1 to 4 were prepared in the same manner as in Example 2-1, with the exception that LPS was used at such a low concentration (0.35 µg/kg) as not to induce inflammation for the wild-type mice, instead of the various concentrations of LPS for groups 2 and 4.

Sera were extracted in the same manner from the mouse models and quantitatively analyzed for the proteins with the aid of the ELISA kit according to the manufacturer's instruction. Blood levels of the circadian clock-controlled inflammatory cytokines IL-6, CXCL1, CCL2, and CCL5, measured in sera of the mice in groups 1 to 4, are summarized in Table 2, below.

**TABLE2**

| Inflammatory cytokine secreted | IL-6 (pg/mL) | CXCL1 (pg/mL) | CCL2 (pg/mL) | CCL5 (pg/mL) | TNF-α (pg/mL) |
|---|---|---|---|---|---|
| Group 1- WT (LPS(-)) | 59.231± 11.121 | 290.073± 47.194 | 49.908± 14.051 | 81.978± 13.271 | 65.792± 9.335 |
| Group 2- WT (LPS(+)) | 60.385± 8.520 | 298.861± 27.363 | 63.494± 17.542 | 108.104± 24.672 | 58.332± 7.341 |
| Group 3- KI/+ (LPS(-)) | 47.048± 11.825 | 252.408± 38.448 | 66.926± 24.473 | 99.095± 28.489 | 64.929± 3.615 |
| Group 4- KI/+ (LPS(+)) | 190.945± 28.265 | 447.010± 13.464 | 263.378± 15.024 | 244.741± 36.921 | 69.896± 7.249 |

In Table 2, the blood levels of the circadian clock-controlled inflammatory cytokines IL-6, CXCL1, CCL2, and CCL5 are expressed as mean ± SEM. When a low concentration of LPS(0.35 µg/kg) which did not cause an inflammatory response in wild-type mice of group 2 was injected, inflammatory cytokines were significantly increased only in Psen2 Alzheimer's disease mouse of group 4, unlike TNF-α.

As shown in FIG. 2b, the lowest LPS concentration (0.35 µg/kg body weight) did not cause inflammation in the wild-type mice, but still remarkably increased blood levels of the circadian clock-controlled inflammatory cytokines (IL-6, CXCL1, CCL2, and CCL5) in the Psen2 mouse model of Alzheimer's disease.

### EXAMPLE 3: Identification of inflammatory aggravation in neuroinflammatory animal model by microglial morphology

Microglial morphology is closely related to the function of microglia, and microglial activation is characterized by cell shape change. To investigate whether the increased production of the circadian clock-controlled cytokines is associated with changes in microglial morphology, an examination was made of microglial shapes in the hippocampus of the wild-type mice and the Psen2 mutation mouse model of Alzheimer's disease by immunohistochemical analysis with an antibody against the microglia-specific marker Iba-1.

For the mice of groups 1 to 4 prepared in Example 2-2, immunohistochemical analysis and confocal analysis were performed. The concentration of LPS injected into groups 2 and 4 was so low (0.35 µg/kg) as not to induce inflammatory response in the wild-type mice.

For immunohistochemical analysis, mice were anesthetized by injection of a mixture of Zoletil (Virbac, 50 mg/kg) and Rompun (Bayer, 10 mg/kg). Then, the mice were perfused with PBS, followed by 4% PFA for fixation. Brains were collected, post-fixed in 4% PFA for 16 hours, transferred to 30% sucrose until they sank to the bottom of the tube, and frozen in a cryoprotective solution. The embedded brains were cut into 50-µm-thick coronal sections, and subjected to an antigen retrieval process at 95°C. Subsequently, the slices were incubated with IBA-1 antibody (1:250) in PBS containing 3% bovine serum albumin for 24 hours at 4°C and then with an appropriate secondary antibody for 2 hours at room temperature. Images were acquired with LSM 7 and LSM 700 confocal laser scanning microscope.

As shown in FIG. 3a, microglia in the hippocampus of the wild-type mice of group 1 had a small cell body with highly ramified processes. Consistent with no induction of cytokine release, a low concentration of LPS did not change the morphology of the hippocampal microglia of the wild-type mice of group 2. On the other hand, hippocampal microglia in the Psen2 mutation mouse model of Alzheimer's disease of group 3 had a round enlarged soma with shorter processes even in the absence of LPS challenge, and these morphological features were furthered by LPS injection, as measured in group 4.

As shown in FIG. 3b, the confocal images of microglia were reconstructed into their 3D morphology and morphological parameters were measured using IMARIS software. Specifically, confocal images were obtained along the entire Z-axis of a randomly selected field, followed by reconstructing 3D images with the aid of IMARIS software (v9.2.1, bitplane AG).

**TABLE3**

| IMARIS Analysis | Dendrite Length (µm) | No. of Dendrite Branch |
|---|---|---|
| Group 1- WT(LPS(-)) | 679.113±38.931 | 88.226±16.285 |
| Group 2- WT(LPS(+)) | 774.000±38.151 | 81.161±13.253 |
| Group 3- KI/+(LPS(-)) | 531.981±31.526 | 44.645±3.416 |
| Group 4- KI/+(LPS(+)) | 420.806±21.694 | 35.871±2.186 |

In Table 3, measurements of dendrite lengths and numbers of dendrite branches are summarized (mean ± SEM).

As can be seen in FIG. 3c, total dendrite length and the number of dendrite terminal points of each microglial cell were lower in the mouse models of Alzheimer's disease of groups 3 and 4 than in the wild-type mice of groups 1 and 2 and further reduced by LPS injection. Therefore, on the basis of morphology, microglial activation was evident in the Psen2 mutation mouse models of Alzheimer's disease at baseline and was further induced by mild LPS injection.

### EXAMPLE 4: Memory Decline in Neuroinflammatory Animal Model

### 4-1. Y-maze assay

To examine spatial learning and memory of the mice in groups 1 to 4 prepared in Example 2-2, a Y-maze assay was carried out 20 hours after LPS injection. Groups 2 and 4 were injected with LPS at the same low concentration ineffective in wild-type mice.

Specifically, the Y-maze was used to evaluate spatial working memory. The assay was conducted in white plastic arms of a Y-shaped maze. A mouse was placed in the center of the maze and was allowed to freely explore the arms for 5 min. The experiment was recorded with EthoVision software (Noldus). The number of arm entries and the number of triads were analyzed to calculate the percentage of alternation by dividing the number of three consecutive arm entries (triads) by the number of possible triads × 100 (total arm entries - 2).

**TABLE 4**

| Y-maze | Alternation % | No. of Arm entry |
|---|---|---|
| Group 1- WT(LPS(-)) | 65.120±4.161 | 16.769±1.574 |
| Group 2- WT(LPS(+)) | 66.124±4.441 | 14.923±1.129 |
| Group 3- KI/+(LPS(-)) | 61.475±4.855 | 15.417±1.209 |
| Group 4- KI/+(LPS(+)) | 42.353±4.137 | 14.200±0.818 |

In Table 4, percentages of alternation and numbers of arm entries of each group in Y-maze are summarized and expressed as mean ± SEM. Data in FIGS. 4a and 4b show that there is no difference in memory performance between the wild-type mice (13 mice) of group 1 and the LPS-injected wild-type mice of group 2 (13 mice) in proportion to inflammatory cytokine release as assayed for the arm alternation in the Y-maze. No differences in memory performance were observed between the groups without LPS injection, that is, the wild-type mice of group 1 and the Psen2 mutation mouse model of Alzheimer's disease of group 3 (12 mice). However, the arm alternation in the Y-maze was significantly declined in the Psen2 mutation mouse model of Alzheimer's disease with LPS injection of group 4 (15 mice). The total number of arm entries was similar across all the groups, indicating normal locomotor functions.

### 4-2. T-maze assay

To further examine learning and memory, the mice of groups 1 to 4 prepared in Example 2-2 were subjected to a T-maze test with a food reward 20 hours after LPS injection. Groups 2 and 4 were injected with LPS at the low concentration (0.35 µg/kg) not inducing inflammatory response in wild-type mice.

Specifically, T-maze was used to evaluate spatial learning and memory with reward alternation. As shown in FIG. 4c, the assay was conducted in white plastic arms of a T-shaped maze. Mice were familiarized with the maze and food reward for 5 min before the test. Then, in the test run, rewards were placed in both arms, and one arm was blocked. Mice were placed at the base and ran to open arms to eat the reward. At the next trial, the previously closed arm was opened. Mice were placed back again at the base and chose one arm. If mice chose the newly opened arm, they were able to eat the reward. If mice incorrectly chose the previously visited arm, they did not get any reward. The number of trials in which the correct arm was visited was expressed as a percentage of total arm entries.

**TABLE 5**

| T-maze | Success rate (%) |
|---|---|
| Group 1- WT(LPS(-)) | 68.831±5.030 |
| Group 2- WT(LPS(+)) | 63.636±3.907 |
| Group 3- KI/+(LPS(-)) | 64.113±7.608 |
| Group 4- KI/+(LPS(+)) | 25.000±3.761 |

In Table 5, success rates of each group in the T-maze test are given as average values (mean ± SEM). Data in FIG. 4d show that there is no difference in learning and memory performance between the wild-type mice (11 mice) of group 1 and the LPS-injected wild-type mice of group 2 (11 mice) in proportion to inflammatory cytokine release. No differences in learning and memory performance were observed between the groups without LPS injection, that is, the wild-type mice of group 1 and the Psen2 mutation mouse model of Alzheimer's disease of group 3 (10 mice). However, the success rates were significantly declined in the Psen2 mutation mouse model of Alzheimer's disease with LPS injection of group 4 (10 mice).

From the data, it was understood that a low concentration of LPS induced a hyperactive immune response and caused memory deficit through the overproduction of clock-controlled cytokines including IL-6 in Psen2^{N141I}/+ mice with Alzheimer's disease, while the same concentration of LPS was innocuous to wild-type mice.

### EXAMPLE 5: Suppression efficacy of drug on neuroinflammation in disease animal

### 5-1: Preparation of murine primary microglia

Primary microglia were obtained from wild-type mice and Psen2^{N141I}/+ mice with Alzheimer's disease. Specifically, brains were excised from neonatal mice 1 to 3 days old and cells were obtained therefrom. The cells were cultured in Dulbecco's modified Eagle's medium (DMEM, Corning) supplemented with 10% heat-inactivated fetal bovine serum (HI-FBS, Hyclone) and 1% penicillin-streptomycin (Hyclone). Primary microglia were isolated in vitro by tapping after 12 days of culturing. Purity of primary microglia was estimated by immunostaining with an antibody against Iba-1, which is a microglial marker.

### 5-2: Assay for production and expression of circadian clock gene-control cytokines in microglia in response to chlorpromazine

Primary microglia obtained in Example 5-1 from the wild-type mice and the Psen2 mutation mouse model of Alzheimer's disease were all treated with LPS (1 µg/mL) for 12 hours. In this regard, the primary microglia were preincubated with or without 0.5 µM chlorpromazine for 30 min before LPS treatment. Anti-inflammatory effects were compared and analyzed between the test groups. Specifically, the primary microglia of Example 5-1 pre-incubated with or without chlorpromazine were classified into: group 5 (WT/LPS(+)/CPZ(-)) and group 6 (WT/LPS(+)/CPZ(+)), respectively, if they were derived from the wild-type mice; and group 7 (KI/+/LPS(+)/CPZ(-)) and group 8 (KI/+/LPS(+)/CPZ(+)), respectively, if they were derived from the Psen2 mutation mouse model of Alzheimer's disease.

In order to qualitatively and quantitatively analyze the circadian clock-controlled cytokines IL-6, TNFα, CCL2, CXCL1, and CCL5, the cell cultures were subjected to ELISA using respective ELISA kits (R&D Systems) according to the manufacturer's instruction.

The culture of each group was measured for microglial mRNA expression levels of the target cytokines by qRT-PCR (quantitative RT-PCR). Specifically, RNA was isolated from the microglia and cDNA was synthesized using an ImProm-II Reverse Transcriptase kit (Promega). PCR primers were commercially available (Cosmo Genetech). qRT-PCR was performed using Taq Polymerase (Invitrogen) and mouse cDNA-specific primers listed in Table 6, below. In addition, TOPreal^{™} qPCR 2 × PreMIX (SYBR Green with low ROX) (Enzynomics) was used. Amplification (50 cycles) was performed for 50 cycles with all primers in a CFX96 Real-Time System (Bio-Rad). Actb was used as the reference genes for normalization.

**TABLE 6**

| **Gene** | **Gene reference number** | **5'- Primer sequence -3'** | |
|---|---|---|---|
| *IL-6* | NM_031168.2 | F | CTGGATATAATCAGGAAATTTGC (SEQ ID NO: 3) |
| | | R | AAATCTTTTACCTCTTGGTTGA (SEQ ID NO: 4) |
| *Cxcl1* | NM_008176.3 | F | CATGGCTGGGATTCACCTCA (SEQ ID NO: 5) |
| | | R | TGAGGTGAATCCCAGCCATG (SEQ ID NO: 6) |
| *Ccl2* | NM_011333.3 | F | GTCCCTGTCATGCTTCTGGG (SEQ ID NO: 7) |
| | | R | CCCCAAGAAGGAATGGGTCC (SEQ ID NO: 8) |
| *Ccl5* | NM_013653.3 | F | CCTCACCATATGGCTCGGAC (SEQ ID NO: 9) |
| | | R | TGCTCCAATCTTGCAGTCGT (SEQ ID NO: 10) |
| *Tnf* | NM_013693.3 | F | CATCTTCTCAAAATTCGAGTGACAA (SEQ ID NO: 11) |
| | | R | TGGGAGTAGACAAGGTACAACCC (SEQ ID NO: 12) |
| *Actb* | NM_007393.5 | F | AGAGGGAAATCGTGCGTGAC (SEQ ID NO: 13) |
| | | R | CAATAGTGATGACCTGGCCGT (SEQ ID NO: 14) |

**TABLE 7**

| Inflammatory cytokine secreted | IL-6 (pg/mL) | CXCL1 (pg/mL) | CCL2 (pg/mL) | CCL5 (pg/mL) | TNF-α (pg/mL) |
|---|---|---|---|---|---|
| Group 5-WT/LPS(+)/CPZ(-) | 2340.109± 150.674 | 963.368± 22.281 | 985.346± 9.691 | 879.119± 31.723 | 1567.490± 97.093 |
| Group 6-WT/LPS(+)/CPZ(+) | 2228.546± 270.079 | 937.727± 46.766 | 948.716± 50.888 | 948.716± 7.882 | 1414.237± 128.292 |
| Group 7-KI/+/LPS(+)/CPZ(-) | 4275.466± 310.119 | 1315.016± 72.985 | 1271.060± 57.569 | 1285.712± 51.932 | 1497.977± 133.153 |
| Group 8-KI/+/LPS(+)/CPZ(+) | 2724.458± 82.940 | 924.907± 87.126 | 734.064± 70.341 | 804.394± 87.093 | 1532.733± 91.847 |

**TABLE8**

| Inflammatory cytokine expression (fold) | *Il-6* | *Cxcl1* | *Ccl2* | *Ccl5* | *Tnf* |
|---|---|---|---|---|---|
| Group 5-WT/LPS(+)/ CPZ(-) | 1.000± 0.052 | 0.975± 0.028 | 1.000± 0.109 | 1.000± 0.023 | 1.000± 0.018 |
| Group 6-WT/LPS(+)/ CPZ(+) | 0.947± 0.032 | 0.968± 0.045 | 0.907± 0.081 | 1.113± .042 | 1.066± 0.054 |
| Group 7-KI/+/LPS(+)/ CPZ(-) | 1.402± 0.015 | 1.436± 0.080 | 1.461± 0.078 | 1.600± 0.063 | 1.080± 0.124 |
| Group 8- KI/+/LPS(+)/ CPZ(+) | 0.538± 0.099 | 0.692± 0.190 | 0.670± 0.128 | 0.730± 0.047 | 1.020± 0.035 |

Expression levels of the circadian clock-controlled inflammatory cytokines, measured in the microglial cell cultures, are depicted in FIG. 5a and summarized in average values (mean ± SEM) in Table 7 while mRNA expression levels of the circadian clock-controlled inflammatory cytokines, measured in the microglial cell cultures, are depicted in FIG. 5b and summarized in average values (mean ± SEM) in Table 8.

Consistent with data from the inflammatory cytokine assay of mouse sera in Example 2-2, data in FIG. 5a shows that levels of the circadian clock-controlled inflammatory cytokines (IL-6, CXCL1, CCL2, and CCL5) were increased in the Psen2 mutation microglia of group 7, compared to the wild-type mice of group 5. From the comparison between the Psen2 mutation microglia of groups 7 and 8, treatment with chlorpromazine at a dose of 0.5 µM was observed to decrease the production of IL-6, CXCL1, CCL2, and CCL5. As detected in groups 5 and 6, the protein levels in wild-type microglia were not affected by the dose of chlorpromazine. Unlike the circadian clock-controlled cytokine, the production of TNF-α was similar across all the groups, with no impacts of chlorpromazine thereon.

As in FIG. 5a, FIG. 5b shows that mRNA levels of the circadian clock-controlled inflammatory cytokines (IL-6, CXCL1, CCL2, and CCL5) were increased in the Psen2 mutation microglia of group 7, compared to the wild-type mice of group 5. From the comparison between the Psen2 mutation microglia of groups 7 and 8, treatment with chlorpromazine at a dose of 0.5 µM was also observed to decrease the mRNA expression of IL-6, CXCL1, CCL2, and CCL5. As detected in groups 5 and 6, the mRNA levels in wild-type microglia were not affected by the dose of chlorpromazine. Unlike the circadian clock-controlled cytokine, the mRNA level of TNF-α was similar across all the groups, with no impacts of chlorpromazine thereon.

### 5-3: Assay for production and expression of circadian cycle-independent cytokine II-1β according to chlorpromazine treatment

In contrast to the circadian clock-controlled cytokines, the production and expression of TNF-α is not affected by chlorpromazine, as discussed in Example 5-2. Likewise, IL-1β, which is also known as a circadian cycle-independent cytokine, was analyzed for production and expression in Example 5-3.

IL-1β is a cytokine released from an activated inflammasome complex in microglia. For secretion of the cytokine, additional treatment with a drug for inducing activation of the inflammasome complex is necessary following LPS-induced inflammation. A primary culture of microglia from the wild-type and the Psen2 mutation mouse model of Alzheimer's disease was pre-incubated with or without chlorpromazine at a dose of 0.5 µM for 30 minutes before three hours of LPS treatment (1 µg/mL) in a fetal bovine serum (HI-FBS)-free medium. Thereafter, the culture was stimulated for 40 minutes with nigericin (10 µM) and ATP (5 mM), followed by performing ELISA with an ELISA kit for IL-1β (R&D Systems) according to the manufacturer's instruction. Application of the test groups thus obtained could divide groups 5 to 8 of Example 5-2 into 10 subgroups according to treatment with nigericin and ATP. The groups further divided are listed in Table 11, below.

After being isolated, the microglial mRNA was analyzed by qRT-PCR as in Example 5-2. The primers used are listed in Table 9.

**TABLE 9**

| **Gene** | **Gene reference number** | **5'- primer sequence -3'** | |
|---|---|---|---|
| *Il-1b* | NM_008361 | F | AGGCCACAGGTATTTTGT (SEQ ID NO: 15) |
| | | R | GCCCATCCTCTGTGACTC (SEQ ID NO: 16) |
| *Actb* | NM_007393.5 | F | AGAGGGAAATCGTGCGTGAC (SEQ ID NO: 17) |
| | | R | CAATAGTGATGACCTGGCCGT (SEQ ID NO: 18) |

In Tables 10 and 11, production and expression levels of IL-1β in primary microglia from the wild-type mice and the Psen2 mutation mouse model of Alzheimer's disease are summarized as average values (mean ± SEM) according to LPS treatment and inflammasome activation for each group.

**TABLE 10**

| Test group | Relative mRNA expression of II-1β (fold) |
|---|---|
| Group 9- WT(LPS(-)) | 1.005±0.080 |
| Group 10-WT(LPS(+)) | 9.576±0.372 |
| Group 11- KI/+(LPS(-)) | 1.005±0.080 |
| Group 12- KI/+(LPS(+)) | 10.025±0.276 |

**TABLE 11**

| Test group | Microglia treated | Nigericin/ ATP treatment | IL-1β secreted (pg/mL) |
|---|---|---|---|
| Group 13 | Group 5-LPS(+)/CPZ(-) | Nig(-)/ATP(-) | 33.411±30.303 |
| Group 14 | Group 5-LPS(+)/CPZ(-) | Nig(+)/ATP(-) | 887.941±72.726 |
| Group 15 | Group 5-LPS(+)/CPZ(+) | Nig(+)/ATP(-) | 857.639±163.634 |
| Group 16 | Group 6-LPS(+)/CPZ(-) | Nig(-)/ATP(+) | 1239.450±36.363 |
| Group 17 | Group 6-LPS(+)/CPZ(+) | Nig(-)/ATP(+) | 1251.571±12.121 |
| Group 18 | Group 7-LPS(+)/CPZ(-) | Nig(-)/ATP(-) | 70.986±67.878 |
| Group 19 | Group 7-LPS(+)/CPZ(-) | Nig(+)/ATP(-) | 771.579±109.089 |
| Group 20 | Group 7-LPS(+)/CPZ(+) | Nig(+)/ATP(-) | 946.122±239.996 |
| Group 21 | Group 8-LPS(+)/CPZ(-) | Nig(-)/ATP(+) | 1237.026±167.270 |
| Group 22 | Group 8-LPS(+)/CPZ(+) | Nig(-)/ATP(+) | 1186.118±130.907 |

FIG. 5c explains that the production and mRNA expression of IL-1β, which is known as a circadian cycle-independent cytokine, is maintained at the same level between microglia from the wild-type mice and the Psen2 mutation mouse model of Alzheimer's disease under LPS-induced inflammation or in the two drugs for inducing inflammasome activation, with no impacts of chlorpromazine on the production of IL-1βacross all the groups.

### EXAMPLE 6: Amyloid beta-induced neuroinflammation and suppressive effect of drug on neuroinflammation in microglia from diseased animal

In Example 5, primary cultures of microglia from the wild-type mice and the Psen2 mutation mouse model of Alzheimer's disease were treated with LPS to induce neuroinflammation and then compared for inhibitory effects of chlorpromazine.

In Example 6, the inhibitory effect of chlorpromazine was analyzed in the primary microglia from the wild-type and the KI/+ mice with Alzheimer's disease after neuroinflammatory induction with fibrilized amyloid beta, which is a direct etiological factor of Alzheimer's disease, instead of the artificially synthesized LPS to more simulate disease conditions.

Specifically, FITC-conjugated amyloid beta 1-42 was prepared according to the follow reference (Cho, M.-H. et al. "Autophagy in microglia degrades extracellular β-amyloid fibrils and regulates the NLRP3 inflammasome." Autophagy 10, 1761-1775 (2014)).

The primary cultures of microglia prepared in Example 5-1 from the Psen2 mutation (KJ/+) mouse model of Alzheimer's disease and the wild-type (WT) mice were directly treated with 4 µM of the fibrilized amyloid beta 1-42 (fAβ42), followed by incubation with or without 0.5 µM chlorpromazine. The cultures thus obtained were quantitatively analyzed for IL-6, TNFα, CCL2, CXCL1, and CCL5 as in Example 5-2, using respective ELISA kits (R&D Systems) according to the manufacturer's instruction.

**TABLE 12**

| Secreted inflammatory cytokine | IL-6 (pg/mL) | CXCL1 (pg/mL) | CCL2 (pg/mL) | CCL5 (pg/mL) | TNF-α (pg/mL) |
|---|---|---|---|---|---|
| Group 23-WT/fAβ42(+)/ CPZ(-) | 1219.285± 60.318 | 1088.070± 174.166 | 1133.372± 58.927 | 1210.244± 41.881 | 1181.114± 105.169 |
| Group 24-WT/fAβ42(+)/CPZ(+) | 1164.200± 39.293 | 1237.646± 31.031 | 1062.537± 72.161 | 1204.562± 27.835 | 1218.952± 126.370 |
| Group 25-KI/+/fAβ42(+)/ CPZ(-) | 1713.141 47.879 | 2135.162± 139.320 | 2100.058± 68.043 | 2465.908± 205.909 | 1305.054± 127.955 |
| Group 26-KI/+/fAβ42(+)/ CPZ(+) | 1336.446± 46.196 | 1528.621± 59.624 | 1645.882± 82.040 | 1818.188± 160.170 | 1352.738± 147.576 |

In Table 12, levels of circadian clock-controlled cytokines in each group after fAβ42-induced neuroinflammation and the inhibition of chlorpromazine against the inflammation are summarized as average values (mean ± SEM).

It was understood from data of FIG. 6 that treatment with fibrilized amyloid beta 1-42 (fAβ42), which is a direct etiological factor of Alzheimer's disease, instead of artificially synthesized LPS, remarkably increased levels of the circadian clock-controlled inflammatory cytokines IL-6, CXCL1, CCL2, and CCL5 in the microglia from the Psen2 mouse model of Alzheimer's disease of groups 23, 25, compared to the microglia from the wild-type mice. Chlorpromazine exhibited an anti-inflammatory effect in the microglia from the Psen2 mutation mouse model of Alzheimer's disease (group 26), but not in the microglia from the wild-type microglia (group 24).

On the other hand, consistent with the data in Example 5, levels of TNF-α were similar between microglia from the wild-type mice and the Psen2 mutation mouse model of Alzheimer's disease, with no impacts of chlorpromazine thereon. From the data thus obtained, it was observed that the Psen2 group with familial Alzheimer's disease undergoes hypersensitive immune responses even to a low immune challenge innocuous to normal groups, with the consequent overexpression of the circadian clock-controlled cytokines. Thus, the neuroinflammation could be reduced even by selectively downregulate the circadian clock-controlled cytokines, suggesting the possibility of development of a dementia therapy that regulates the Psen2 familial dementia-specific, overactivated inflammation.

### EXAMPLE 7: Drug-induced restoration of REV-ERBα expression in disease model

After being cultured in the same manner as in Example 5-1, primary microglia from the wild-type mice and the Psen2 mouse model of Alzheimer's disease were used in the test without LPS treatment. Effects were analyzed in the experimental group not treated with chlorpromazine on the obtained microglia and the experimental group treated with chlorpromazine at a dose of 0.5 µM.

The mRNA expression of *Nr1d1,* which encodes REV-ERBα known as a circadian rhythm-associated protein regulating the circadian clock-controlled inflammatory cytokines (IL-6, CXCL1, CCL2, and CCL5) identified in the foregoing examples, was analyzed using qRT-PCR in the same manner as in Example 5. Primer sets for this qRT-PCR are listed in Table 13, below.

**TABLE 13**

| **Gene** | **Gene reference number** | **5'- primer sequence -3'** | |
|---|---|---|---|
| *Nr1d1* | NM_145434.4 | F | TGCTGGCATGTCCCATGAAC (SEQ ID NO: 19) |
| | | R | GACTGTAGGTTGTGCGGCTC (SEQ ID NO: 20) |
| *Actb* | NM_007393.5 | F | AGAGGGAAATCGTGCGTGAC (SEQ ID NO: 21) |
| | | R | CAATAGTGATGACCTGGCCGT (SEQ ID NO: 22) |

**TABLE 14**

| Test group | *Nr1d1* mRNA expression (fold) | REV-ERBα protein expression (fold) |
|---|---|---|
| Group 27-WT/CPZ(-) | 1.000±0.035 | 1.000±0.029 |
| Group 28-WT/CPZ(+) | 0.860±0.040 | 0.956±0.096 |
| Group 29-KI/+/CPZ(-) | 0.333±0.014 | 0.596±0.014 |
| Group 30- KI/+/CPZ(+) | 0.976±0.094 | 0.882±0.032 |

In Table 14, mRNA expression levels of *Nrldl* gene in primary microglia from the wild-type and the Psen2 mutation mouse model of Alzheimer's disease are summarized as average values (mean ± SEM), along with expression levels of its protein REV-ERBα, according to chlorpromazine treatment.

mRNA expression levels of *Nr1d1*, which encodes REV-ERBα, are depicted in FIG. 7a. Compared to microglia from the wild-type mice of group 27, a significant reduction of mRNA expression levels of *Nr1d1* was detected in microglia from the Psen2 mutation mouse model of Alzheimer's disease of group 29. As observed in the Psen2 mutation mouse model of Alzheimer's disease of group 30, the expression level was restored to the level in the wild-type group by treatment with chlorpromazine at a dose of 0.5 µM, which was ineffective in the wild-type microglia.

For analysis of REV-ERBα protein expression levels, western blotting was carried out. Specifically, primary microglia were lysed in a 1% Triton X-100 lysis buffer (1% Triton X-100, 250 mM sucrose, 1 mM EDTA, 1 mM phenylmethylsulfonyl fluoride, and 50 mM NaCl in 20 mM Tris-HCl, pH 7.4) with 1× protease and phosphatase inhibitors (Thermo Fisher Scientific) and 0.1 M dithiothreitol (Sigma-Aldrich). The cell lysates were separated by sodium dodecyl sulfate-polyacrylamide gel electrophoresis and transferred to polyvinylidene fluoride membranes. The membranes were incubated with the appropriate primary antibodies, and bound antibodies were detected by species-specific, horseradish peroxidase-conjugated secondary antibodies. Chemiluminescence detection was performed to analyze the protein bands of interest.

Consistent with the mRNA expression level data in FIG. 7a, the western blotting analysis results, depicted in FIG. 7b, show that the microglia from the Psen2 mutation mouse model of Alzheimer's disease of group 29 significantly decreased in the expression level of REV-ERBα protein, compared to those from the wild-type mice of group 27. The expression level in microglia from the Psen2 mutation mouse model of Alzheimer's disease of group 30 was restored to the level in the wild-type group by treatment with chlorpromazine at a dose of 0.5 µM. And treatment of chlorpromazine had no effect on wild-type microglia in group 28.

Collectively, the data indicate that the expression level of REV-ERBα, which is known as a circadian rhythm-associated protein regulating the circadian clock-controlled inflammatory cytokines (IL-6, CXCL1, CCL2, and CCL5), was remarkably decreased in the Psen2^{N141I} mutation mouse model of Alzheimer's disease and chlorpromazine can restore the level.

### EXAMPLE 8: Assay for effect of REV-ERBα agonist in diseased animal

For use in this assay, primary microglia were prepared in the same manner as in Example 5-1 from the wild-type mice and the Psen2 mutation mouse model of Alzheimer's disease.

Cultures of primary microglia from the wild-type mice and the Psen2 mutation mouse model of Alzheimer's disease were treated with LPS (1 µg/mL) for 2, 6, 12, and 24 hours after incubation with or without 10 µM SR9009, known as a REV-ERBα agonist. The efficacy of SR9009 was evaluated by analyzing IL-6 at protein and mRNA expression levels according to post-LPS treatment time (2, 6, 12, and 24 hours) in the same ELISA and qRT-PCR as in Example 5.

**TABLE 15**

| IL-6 secreted (pg/mL) | 2 h | 6 h | 12 h | 24 h |
|---|---|---|---|---|
| Group 31-WT/LPS(+)/ SR9009(-) | 29.054± 5.531 | 532.117± 48.062 | 762.167± 88.754 | 1256.895± 102.482 |
| Group 32-WT/LPS(+)/ SR9009(+) | 24.467± 1.529 | 85.630± 2.648 | 361.583± 1.417 | 555.053± 33.535 |
| Group 33-KI/+/LPS(+)/ SR9009(-) | 32.112± 6.665 | 704.289± 66.593 | 1224.309± 36.113 | 1605.522± 101.381 |
| Group 34- KI/+/LPS(+)/ SR9009(+) | 38.229± 1.529 | 332.114± 18.043 | 1146.560± 70.216 | 1657.510± 36.152 |

**TABLE 16**

| IL-6 mRNA expression level (fold) | 2 h | 6 h | 12 h | 24 h |
|---|---|---|---|---|
| Group 31-WT/LPS(+)/ SR9009(-) | 1.000± 0.134 | 5.140± 0.530 | 6.061± 0.146 | 9.933± 0.385 |
| Group 32-WT/LPS(+)/ SR9009(+) | 0.456± 0.050 | 1.098± 0.088 | 1.129± 0.181 | 2.150± 0.058 |
| Group 33-KI/+/LPS(+)/ SR9009(-) | 1.031± 0.004 | 8.773± 0.354 | 8.956± 0.504 | 13.522± 0.740 |
| Group 34- KI/+/LPS(+)/ SR9009(+) | 1.134± 0.010 | 7.810± 0.878 | 8.512± 0.297 | 11.141± 0.282 |

In Tables 15 and 16, protein and mRNA expression levels of IL-6 in primary microglia from the wild-type mice and the Psen2 mutation mouse model of Alzheimer's disease in response to treatment with chlorpromazine are summarized as average values (mean ± SEM), respectively.

In FIG. 8a, SR9009 repressed the production of IL-6 in group 32 treated with SR9009 compared to group 31 according to time (2, 6, 12, and 24 hours) in primary culture wild-type microglia, but compared IL-6 which is overexpressed in microglia derived from Psen2 mutation Alzheimer's disease mouse model of group 33, it cannot repress thereof in SR9009-treated group 4.

In FIG. 8b, SR9009 repressed the mRNA expression of IL-6 in group 32 treated with SR9009 compared to group 31 according to time (2, 6, 12, and 24 hours) in the primary wild-type microglia, but compared to IL-6 which is overexpressed in microglia derived from Psen2 mutant Alzheimer's disease mouse model of group 33, it cannot repress thereof in SR9009-treated group 34.

Thus, the anti-inflammatory activity of SR9009, known as a REV-ERBα agonist, does not work in the microglia from the Psen2^{N141I} mouse model of Alzheimer's disease. From the data, it can be seen that the restoration of REV-ERBα expression must be preceded in order to repress the excessive neuroinflammatory response caused by the Psen2^{N141I} mutation. Also, the data suggest that the REV-ERBα agonist, although exhibiting anti-inflammatory effects in normal microglia, may not have any therapy effect for Psen2 familial dementia.

### EXAMPLE 9: Proving that a decreased expression of REV-ERBα in microglia of diseased animal is the cause of overexpression of Circadian Clock-Controlled Cytokines

### 9-1: REV-ERBα knockdown in microglia from wild-type mice

For use in preparing REV-ERBα-knockdown microglia, sh*Nr1d1* (22747) was purchased from Addgene. Lentiviruses were produced using a transfer vector (PLKO.1-shNr1d1), a packaging vector (psPAX2, Addgene), and a VSV-G envelope-expressing vector (PM2). Three days after transfection of the vectors into HEK293T cells, the supernatant was harvested and ultracentrifuged at 25,000 × g for 2 hours in Optima XPN-100 (Beckman Coulter) for virus concentration. Primary microglia were infected with the lentiviruses in the presence of hexadimethrine bromide (8 µg/mL). eGFP expression was monitored using fluorescence microscopy after 72 h to estimate the lentiviral transduction efficiency.

As in Example 5-1, the primary microglia from the wild-type mice were used in the experiment. The knockdown of REV-ERBα was induced in the microglia from the wild-type mice by expressing shNr1d1 through the lentivirus as stated in the foregoing.

In FIG. 9a, the knockdown of REV-ERBα was successfully conducted in the primary microglia from the wild-type mice as analyzed by the same western blotting as in Example 7.

FIG. 9b shows production of the inflammatory cytokines IL-6, CXCL1, CCL2, CCL5, and TNF-α in the normal wild-type microglia and the REV-ERBα knocked-down wild-type microglia after LPS (1µg/mL) treatment for 12 hours as measured by the same ELISA as in Example 5. Consistent with the LPS treatment in microglia from the Psen2 mutation mouse model of Alzheimer's disease, the downregulation of REV-ERBα elevated the production of the cytokines IL-6, CXCL1, CCL2, and CCL5, except for TNF-α.

FIG. 9c shows mRNA expression of the inflammatory cytokines IL-6, CXCL1, CCL2, CCL5, and TNF-α in the normal wild-type microglia and the REV-ERBα knocked-down wild-type microglia after LPS (1µg/mL) treatment for 12 hours as measured by the same qRT-PCR as in Example 5. Consistent with the LPS treatment in microglia from the Psen2 mutation mouse model of Alzheimer's disease, the downregulation of REV-ERBα elevated the mRNA expression of the cytokines IL-6, CXCL1, CCL2, and CCL5, except for TNF-α.

### 9-2: Overexpression of REV-ERBα in Microglia from Psen2 Mutation Mouse Model of Alzheimer's disease

For use in preparing microglia overexpressing REV-ERBα, human *NR1D1* cDNA was cloned into a PLJM1-EGFP vector to generate lentiviruses. Lentiviruses were produced using a transfer vector (PLKO.1-shNr1d1) a packaging vector (psPAX2, Addgene), and a VSV-G envelope-expressing vector (PM2). Subsequent procedures were carried out in the same manner as in Example 9-1.

As in Example 5-1, the primary microglia from the Psen2 mutation mouse model of Alzheimer's disease were used in the experiment. *NR1D1* was overexpressed in the microglia from the Psen2 mutation mouse model in the same manner as in Example 9-1 through the lentivirus.

In FIG. 9d, REV-ERBα overexpression in the primary microglia from the Psen2 mutation mouse model of Alzheimer's disease increased the amount of REV-ERBα protein was confirmed the same western blotting as in Example 7.

FIG. 9e shows production of the inflammatory cytokines IL-6, CXCL1, CCL2, CCL5, and TNF-α in microglia from the Psen2^{N141I} mutant mouse model of Alzheimer's disease with or without REV-ERBα overexpression after LPS (1µg/mL) treatment for 12 hours as measured by the same ELISA as in Example 5. Consistent with the LPS treatment in microglia from the wild-type mice, the overexpression of REV-ERBα remarkably reduced the production of the cytokines IL-6, CXCL1, CCL2, and CCL5, except for TNF-α.

FIG. 9f shows mRNA expression of the inflammatory cytokines IL-6, CXCL1, CCL2, CCL5, and TNF-α in microglia from the Psen2^{N141I} mutant mouse model of Alzheimer's disease with or without REV-ERBα overexpression after LPS (1µg/mL) treatment for 12 hours, as measured by the same qRT-PCR as in Example 5. Consistent with the LPS treatment in microglia from the wild-type mice, the overexpression of REV-ERBα in microglia from the Psen2^{N141I} mouse model of Alzheimer's disease remarkably reduced the mRNA expression of the cytokines IL-6, CXCL1, CCL2, and CCL5, except for TNF-α.

Taken together, the data obtained above indicate that the downregulated expression of REV-ERBα provokes overexpression of the circadian clock-controlled cytokines in microglia from the Psen2 mutation mouse model of Alzheimer's disease.

### EXAMPLE 10: Characterization of Psen2 Familial Alzheimer's Disease through Comparison with 5xFAD animal model of Alzheimer's disease

### 10-1: 5xFAD mouse model

Advantage was taken of the SXFAD mouse model, one of animal models used for study on Alzheimer's disease, which expresses *APP* and *PSEN1* genes with a total of 5 AD-related mutations (APP; Swedish (K670N/M671L), Florida (I716V), and London (V717I) mutations and PSEN1; M146L and L286V mutations). Expression of the *APP* and *PSEN1* mutation genes are driven by the Thy1 (mature neuron-specific marker) promoter and even the semi-conjugated mice exhibit severe amyloid pathology and behavior impairment (Jawhar S. et al. "Motor deficits, neuron loss, and reduced anxiety coinciding with axonal degeneration and intraneuronal Aβ aggregation in the SXFAD mouse model of Alzheimer's disease." Neurobiology of Aging 196. e29-40 (2012))

### 10-2: Characterization of Psen2 familial Alzheimer's disease

For use in this experiment, primary microglia were obtained from the wild-type mice and the mouse model of 5xFAD Alzheimer's disease as in Example 5-1. *Nrldl* mRNA expression levels were compared between the microglia from the wild-type mice and the mouse model of 5xFAD Alzheimer's disease by qRT-PCR using the *Nrldl* primers of Table 13 as in Example 7. In Table 17, *Nr1d1* mRNA expression levels in microglia from the wild-type mice and the mouse model of 5xFAD Alzheimer's disease are summarized as average values (mean ± SEM).

**TABLE 17**

| Test group | Nr1d1 mRNA expression level (fold) |
|---|---|
| Group 35-wild-type | 0.978±0.092 |
| Group 36-5xFAD Alzheimer's disease model | 1.031±0.168 |

In addition, blood was taken from the cheeks of the wild-type mice and the 5xFAD mouse model of Alzheimer's disease, and sera were obtained by centrifugation and measured for expression levels of inflammatory cytokine, as in Example 2. In Table 18 below, expression levels of the inflammatory cytokines IL-6 and TNF-α in microglia from the wild-type mice and the mouse model of 5xFAD Alzheimer's disease are summarized as average values (mean ± SEM).

**TABLE 18**

| Test group | IL-6 protein (pg/mL) | TNF-α protein (pg/mL) |
|---|---|---|
| Group 35-wild-type | 98.542±7.451 | 52.864±9.150 |
| Group 36-5xFAD Alzheimer's disease model | 101.563±9.674 | 55.560±5.392 |

As shown in FIG. 10a, *Nrldl* mRNA expression levels were same between primary microglia from the wild-type mice and the mouse model of 5xFAD Alzheimer's disease. In FIG. 10b, there is no difference in blood levels of inflammatory cytokines IL-6 and TNF-α between the wild-type mice and the mouse model of 5xFAD Alzheimer's disease. These data obtained from the 5xFAD mice used as a mouse model expressing the *APP* and *PSEN1* gene mutations indicate that the hyperactive immune response due to the downregulated expression of REV-ERBα may be responsible for a feature of the Psen2^{N141I} familial Alzheimer's disease.

### EXAMPLE 11: Comparison of effects of drug on expression of in vivo circadian cytokines and memory recovery of disease animal model

### 11-1: Drug effect on LPS-induced neuroinflammation in disease animal model

The wild-type mice and the Psen2 mutation mouse model of Alzheimer's disease obtained in Example 1 were intraperitoneally injected with chlorpromazine (0.25 mg/body weight kg) at 14:00 and then with such a low concentration of LPS (0.35 µg/kg) as to be ineffective for inflammation in wild-type mice at 18:00 (Zeitgeber time light-on at 11:00 and 07:00). After 20 hours, the mice were monitored for inflammatory responses and memory behavior. The concentration of chlorpromazine (0.25 mg/body weight kg) used in this assay is tens- to hundreds-fold lower than those conventionally used as an antipsychotic (25 mg/injection, 3 times/day). The strategy is schematically illustrated in FIG. 11a.

After the experiment conducted as in the scheme of FIG. 11a, the wild-type and the Psen2^{N141I} KI/+ Alzheimer's disease mice, both intraperitoneally injected with LPS, were analyzed for inflammatory response according to chlorpromazine treatment as measured for serum levels of the circadian clock-controlled cytokines (IL-6, CXCL1, CCL2, and CCL5) by ELISA as in Example 2. In Table 19 below, measurements of serum levels of the circadian clock inflammatory cytokines in the wild-type mice and the Psen2 mutation mouse model of Alzheimer's disease are summarized as average values (mean ± SEM).

**TABLE 19**

| Inflammatory cytokine secreted | IL-6 (pg/mL) | CXCL1 (pg/mL) | CCL2 (pg/mL) | CCL5 (pg/mL) | TNF-α (pg/mL) |
|---|---|---|---|---|---|
| Group 37-wild-type/ LPS(+)/CPZ(-) | 72.478± 13.597 | 182.149± 27.667 | 103.699± 6.197 | 98.485± 3.388 | 64.570± 9.209 |
| Group 38-wild-type/ LPS (+)/CPZ(+) | 68.214± 14.143 | 172.313± 27.437 | 117.032± 14.132 | 80.808± 5.758 | 62.634± 5.202 |
| Group 39-Alzheimer's disease model/ LPS (+)/CPZ(-) | 185.214± 15.991 | 365.750± 70.334 | 257.138± 19.910 | 247.476± 14.594 | 66.919± 9.052 |
| Group 40-Alzheimer's disease model/ LPS (+)/CPZ(+) | 44.280± 1.527 | 173.999± 14.795 | 107.403± 11.219 | 93.240± 5.245 | 71.248± 8.078 |

As can be understood from Table 19 and FIG. 11b, the injection of chlorpromazine did not affect the production of the circadian clock-controlled cytokines in the wild-type mice of groups 37 and 38 which were both injected with LPS at such a low concentration (0.35 µg/kg) as to be ineffective for inflammation induction in wild-type mice. On the other hand, even the low concentration of LPS induced the overexpression of the circadian clock-controlled cytokines in the Psen2 mutation mouse model of Alzheimer's disease of group 39, but chlorpromazine remarkably repressed the production of the cytokines in the Psen2 mutation mice of Alzheimer's disease injected with chlorpromazine (group 40).

### 11-2: Drug effect on memory decline caused by LPS-induced inflammation in diseased animal model

An examination was made to see whether LPS-induced inflammation causes memory decline and chlorpromazine reverses the LPS-induced inflammation in disease animal models. To analyze memory and behavior performance, the Y-maze test as in Example 4 was conducted. Table 20 summarizes Y-maze measurements (mean ± SEM) to evaluate influences of chlorpromazine against LPS-induced memory decline in the wild-type mice and the Psen2 mutation mouse model of Alzheimer's disease.

**TABLE 20**

| Y-maze | Alternation % | No. of Arm entry |
|---|---|---|
| Group 41-wild-type/LPS(-)/CPZ(-) | 66.799±3.743 | 19.286±1.924 |
| Group 42-wild-type/LPS(-)/CPZ(+) | 66.667±4.681 | 20.167±1.537 |
| Group 43-wild-type/LPS(+)/CPZ(-) | 65.563±5.049 | 19.714±1.063 |
| Group 44-wild-type/LPS(+)/CPZ(+) | 64.333±3.175 | 17.889±1.136 |
| Group 45-Alzheimer's disease model/LPS(-)/CPZ(-) | 69.468±3.555 | 17.286±1.700 |
| Group 46-Alzheimer's disease model/LPS(-)/CPZ(+) | 77.714±4.699 | 18.286±1.063 |
| Group 47-Alzheimer's disease model/LPS(+)/CPZ(-) | 35.731±5.865 | 15.600±1.147 |
| Group 48-Alzheimer's disease model/LPS(+)/CPZ(+) | 72.000±3.964 | 16.500±1.368 |

As shown in FIG. 11c for arm alternation in Y-maze, all the wild-type mice injected with chlorpromazine alone (group 42) the wild-type mice injected with LPS alone (group 43), and the mice injected with both LPS and chlorpromazine (group 44) exhibited normal memory performance as in the wild-type mice injected with none of the drugs (group 41). Also, no significant different memory performance was found between the Psen2 mutation Alzheimer's disease mice with no drug treatment of group 45 (7 mice) and the Psen2 mutation Alzheimer's disease mice injected with chlorpromazine alone of group 45 (7 mice). In contrast, the LPS-injected Psen2 mutation mouse model of Alzheimer's disease of group 47 (9 mice) suffered from significant memory decline, and chlorpromazine injection made a sufficient recovery from the LPS-induced memory decline in the LPS-injected Psen2 mutation mouse model of Alzheimer's disease of group 48.

In FIG. 11d, similar total numbers of arm entries were measured across all the groups, indicating normal locomotor activity.

Taken together, the data obtained above demonstrate that in vivo injection of chlorpromazine into Psen2^{N141I} Alzheimer's disease mice can recover hyperactive neuroinflammation and memory deficits.

## Claims

1. A pharmaceutical composition for prevention or treatment of a neuroinflammatory disease in a subject, wherein the pharmaceutical composition comprises chlorpromazine or a pharmaceutically acceptable salt thereof.

2. The pharmaceutical composition of claim 1, wherein the disease is **characterized by** overexpression of a circadian clock-controlled inflammatory cytokine, compared to a normal subj ect.

3. The pharmaceutical composition of claim 2, wherein the circadian clock-controlled inflammatory cytokine is at least one selected from the group consisting of IL-6, CXCL1, CCL2, and CCL5.

4. The pharmaceutical composition of claim 2, wherein the disease is **characterized by** overexpression of a circadian clock-controlled inflammatory cytokine in glial cell of the central nervous system, compared to a normal subject.

5. The pharmaceutical composition of claim 1, wherein the disease is **characterized by** low expression of Nuclear Receptor Subfamily 1 Group D Member 1 (Nrld1) gene, compared to a normal subject.

6. The pharmaceutical composition of claim 1, wherein the disease is a disease comprising Presenilin 2 (PSEN2) mutation.

7. The pharmaceutical composition of claim 6, wherein the PSEN2 mutation comprises Psen2^{N141I} and at least one selected from the group consisting of A85V, N141Y, M174I, G212V, A237V, M239I, and M239V.

8. The pharmaceutical composition of claim 1, wherein the neuroinflammatory disease is selected from the group consisting of multiple sclerosis, neuroblastoma, stroke, Alzheimer's disease, Parkinson's disease, Lou Gehrig's disease, Huntington's disease, Creutzfeldt-Jakob disease, post-traumatic stress disorder, depression, schizophrenia, neuropathic pain, and amyotrophic lateral sclerosis.

9. The pharmaceutical composition of claim 8, wherein the disease is Alzheimer's disease with a mutation in a presenilin 2 gene or dementia related to Alzheimer's disease with a mutation in a presenilin 2 gene.

10. The pharmaceutical composition of claim 8, wherein the disease is familial Alzheimer's disease.

11. The pharmaceutical composition of claim 1, wherein the subject is **characterized by** at least one selected from the group consisting of a mutation in a presenilin 2 gene, overexpression of a circadian clock-controlled cytokine, and downregulated expression of a Nr1d1 gene.

12. The pharmaceutical composition of claim 2, wherein the composition suppresses overexpression of the circadian clock-controlled inflammatory cytokine compared to a normal subject.

13. The pharmaceutical composition of claim 1, wherein the composition does not suppress overexpression of TNF-alpha.

14. The pharmaceutical composition of claim 1, wherein the composition is **characterized by** at least one selected from the group consisting of suppression of neuroinflammation, inhibition of overexpression of a circadian clock-controlled inflammatory cytokine, restoration of REV-ERVα expression, memory restoration, and cognitive improvement.

15. The pharmaceutical composition of claim 1, wherein the prevention or treatment is carried out by administering chlorpromazine or a pharmaceutically acceptable salt thereof in a sufficient amount enough to reduce neuroinflammation.

16. The pharmaceutical composition of claim 1, wherein the chlorpromazine or a pharmaceutically acceptable salt thereof is 0.0001 to 1.0 mg/kg/day, based on a form of chlorpromazine free base.

17. A method for preventing or treating a neuroinflammatory disease, comprising a step of administering chlorpromazine or a pharmaceutically acceptable salt thereof to a subject in need thereof.

18. Chlorpromazine or a pharmaceutically acceptable salt thereof for use in preventing or treating a neuroinflammatory disease in a subject.
